(19) 

**Europäisches Patentamt**
**European Patent Office**
**Office européen des brevets**

(11) **EP 3 934 423 B1**

(12) **EUROPEAN PATENT SPECIFICATION**

(45) Date of publication and mention
of the grant of the patent:
**20.08.2025 Bulletin 2025/34**

(21) Application number: **20707466.7**

(22) Date of filing: **06.03.2020**

(51) International Patent Classification (IPC):
***A01N 25/00*** *(2006.01)* ***A61K 49/00*** *(2006.01)*
***G01N 33/60*** *(2006.01)*

(52) Cooperative Patent Classification (CPC):
**A01N 25/00; G01N 33/60;** G01N 2570/00

(86) International application number:
**PCT/EP2020/056006**

(87) International publication number:
**WO 2020/182652 (17.09.2020 Gazette 2020/38)**

(54) **METABOLIC ANALYSIS METHOD**

STOFFWECHSELANALYSEVERFAHREN

PROCÉDÉ D'ANALYSE MÉTABOLIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **08.03.2019 EP 19161645**

(43) Date of publication of application:
**12.01.2022 Bulletin 2022/02**

(73) Proprietor: **BASF SE**
**67056 Ludwigshafen am Rhein (DE)**

(72) Inventors:
• **STARCK, Veronique**
**68623 Lampertheim (DE)**
• **HENDRIKS, Janneke**
**10589 Berlin (DE)**
• **DRIEMERT, Peter**
**10589 Berlin (DE)**
• **HEROLD, Michael**
**10589 Berlin (DE)**
• **MENTZEL, Tobias**
**68169 Mannheim (DE)**
• **CAMPE, Ruth**
**67117 Limburgerhof (DE)**
• **TRESCH, Stefan**
**67056 Ludwigshafen (DE)**
• **FUCHS, Regine**
**10589 Berlin (DE)**

• **HILLER, Karsten**
**38106 Braunschweig (DE)**
• **DUDEK, Christian-Alexander**
**38106 Braunschweig (DE)**

(74) Representative: **BASF IP Association**
**BASF SE**
**GBI - Z078**
**67056 Ludwigshafen (DE)**

(56) References cited:
WO-A1-2005/051434      WO-A1-2005/059556
WO-A2-2005/081943      WO-A2-2005/094327
US-A1- 2003 180 800      US-A1- 2005 281 745

• TURNER S M ET AL: "EMERGING
APPLICATIONS OF KINETIC BIOMARKERS IN
PRECLINICAL AND CLINICAL DRUG
DEVELOPMENT", CURRENT OPINION IN DRUG
DISCOVERY AND DEVELOPMENT, CURRENT
DRUGS, LONDON, GB, vol. 8, no. 1, 1 January
2005 (2005-01-01), pages 115 - 126, XP009052135,
ISSN: 1367-6733
• TURNER ET AL: "Stable isotopes, mass
spectrometry, and molecular fluxes:
Applications to toxicology", JOURNAL OF
PHARMACOLOGICAL AND TOXICOLOGICAL
METHODS, ELSEVIER, NEW YORK, NY, US, vol.
53, no. 1, 1 January 2006 (2006-01-01), pages 75 -
85, XP025015044, ISSN: 1056-8719, [retrieved on
20060101], DOI: 10.1016/J.VASCN.2005.08.001

EP 3 934 423 B1

## Description

### Introduction

It is estimated that the global population will increase from 7.3 billion in 2015 to 9.7 billion in 2050. In order to satisfy this increase in population, the UNFAO has been calculated that world food production will need to raise by approximately 70%.

Improvements in agricultural production can be achieved using a variety of different methods. For example, high yield varieties of animal or plant foods, improved mechanization processes and better fertilizers are all means by which productivity can be enhanced.

One important factor in agricultural improvements is the use of pesticides. A pesticide can be considered as any substance or mixture of substances intended for preventing, destroying, or controlling any pest. Numerous types of pesticides exist, including fungicides, herbicides, insecticides and nematicides. They provide important benefits to food producers by preventing crop losses to insects and other pests. Not only does this lead to an increase in overall food production by virtue of improved yield, there is also a benefit to the consumer by lowering the costs of the produced food.

As can be appreciated, pest resistance can result from the use of pesticides. Pest species evolve pesticide resistance via natural selection: the most resistant specimens survive and pass on their acquired heritable traits to their offspring. It has been reported that instances of pest resistance are on the increase.

Pesticides typically work by affecting one or more biological processes within the target organism. The specific mechanism by which the pesticide works is called 'mode of action'. This can be an understanding as to which biological processes are affected by the pesticide, for example, by disrupting certain biosynthetic pathways or by the inhibition of vital enzymes within the target. Since pesticide resistance can arise by alterations to the target for the pesticide, it is important to identify new pesticides which have different mode of actions to current pesticide agents. Hence the identification of the mode of action of potential pesticide compounds is an important stage in the development of these products.

"Screening leads" is a term commonly used in pesticide (and medicinal) product development programs to generally relate to compounds which display encouraging potency and specificity. In additional to these properties, approximately half of the promising compounds can have a 'mode of action' which is not elucidated easily. In order to select which screening lead compounds to progress into further development, it is common practice to apply 'mode of action' analysis to identify compounds which have new modes of action, since they potentially represent new pesticide classes and thus can provide an advantage in alleviating pesticide resistance. However, elucidating a new mode of action for a screening lead compound can be difficult since there are only a limited number of methods available for this purpose.

WO 2005/051434 A1 describes a high-throughput screening of test compounds for actions on molecular flux rates in metabolic pathways comprising exposing compounds to a living system, administering an isotope-labelled substrate, measuring the content, rate of incorporation and/or pattern or rate of change in content and/or pattern of isotope labelling of targeted molecules, and comparing molecular flux rates in living systems administered or not administered with said test compounds. Mass spectrometry is used for the identification and quantification of metabolites. This document proposes a method that determines how a targeted molecule of interest is affected by a test compound.

To discover new modes of action, a range of molecular, biochemical and physiological in vitro screenings or 'omics'-based profiling approaches are currently used, including protein-protein and protein-compound interaction assays (both in vitro and in vivo) and hip-hop assays. However, these methods are based on previous knowledge of the likely target molecules the screening lead compounds interact with, i.e. they rely on existing known 'mode-of-action' studies, while it is likely undiscovered mode of actions may be located in other, previously unaffected metabolic pathways.

Hence it can be appreciated that there is a need to identify the mode of action of screening leads in pesticide development.

### Summary of the invention

Here we present a non-targeted metabolomics approach for mode-of-action identification.

A first aspect of the invention provides a method of characterising the mode of action of a test compound comprising:

a) A first biological sample and a second biological sample, wherein there is at least one isotopic difference between the samples, and wherein the first and second biological samples are isolated from a living system exposed to the test compound;

b) A third biological sample and a fourth biological sample, wherein there is at least one isotopic difference between the samples, and wherein the third and fourth biological samples are isolated from a living system not exposed to the test compound;

c) Performing chromatograph and mass spectrometry analysis of metabolites extracted from the biological samples to provide mass spectra data;

d) Calculating the mass isotopomer distribution for the metabolites from the mass spectra;

e) Identifying metabolites that have a significant different isotopomer distribution when the living system was exposed to the test compound to the isotopomer distribution when the living system was not exposed to the test compound.

For this purpose, separate populations of the living systems are done under control and treatment conditions, using an unlabeled and labeled isotope source materials.

The measurement of the isotopomer distribution (~enrichment) of each metabolite for the populations can then reveal the effect of the test compounds on the metabolism in that population. By examining the profile of known metabolites it is the possible to link the test compound to specific metabolic processes and hence characterize the mode of action of that compound.

An embodiment of the invention is wherein the mass spectra data provided in step c) is deconvoluted and metabolites subsequently identified from the mass spectra by data comparison with a reference library.

A further embodiment of the invention is wherein the mass isotopomer distribution generated by step d) is determined by: calculating the retention indices and deconvolution of the mass spectral data; pairing the deconvoluted mass spectra data across samples and identifying metabolites according to isotope incorporation; identification of those metabolites based on similarity of spectra, retention time and/or retention index by comparison to a reference library; calculation of the mass isotopomer distribution of the metabolites.

A further embodiment of the invention is wherein step e) comprises comparing the mass isotopomer distribution between metabolites having at least one isotopic difference and identifying those metabolites which have a statistically different deviation between the biological samples isolated from a living system exposed to the test compound and the biological samples isolated from a living system not exposed to the test compound.

A further embodiment of the invention is wherein the mode of action of the test compound is identified according to the metabolites identified from step e).

A further embodiment of the invention is wherein living system incorporates an isotopic label from an isotope-labeled substrate.

A further embodiment of the invention is wherein the living system incorporates an isotopic label from an isotope-labeled substrate.

A further embodiment of the invention is wherein the isotope-labeled substrate is chosen from $^2H_2O$, $H_2\,^{18}O$, $^2H$-glucose, $^2H$-labeled amino acids, $^2H$-labeled organic molecules, $^{13}C$-labeled organic molecules, $^{13}C$-labeled glycerol, $^{13}CO_2$, $^{15}N$-labeled organic molecules, $^3H_2O$, $^3H$-labeled glucose, $^3H$-labeled amino acids, $^3H$-labeled organic molecules.

A further embodiment of the invention is wherein the living system is a plant, fungus, virus, bacteria, invertebrate or vertebrate.

A further embodiment of the invention is wherein the living system is a plant and the isotope-labeled substrate is $^{13}CO_2$

A further embodiment of the invention is wherein the living system is a fungus and the isotope-labeled substrate is $^{13}C$-labeled organic molecules.

A further embodiment of the invention is wherein the living system is exposed to the isotope-labeled substrate for between 10 mins and 48 hours.

A further embodiment of the invention is wherein the living system is exposed to the test substrate for between 10 mins and 48 hours.

A further embodiment of the invention is wherein the test compound is a small molecule or a biological factor.

## Definitions and Description of the invention

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

Further, as used in the following, the terms "preferably", "more preferably", "most preferably", "particularly", "more particularly", "specifically", "more specifically" or similar terms are used in conjunction with optional features, without restricting further possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding further embodiments of the invention, without any restrictions regarding the scope of the invention and without any restriction regarding the possibility of combining the features introduced in such way with other optional or non-optional features of the invention. Moreover, if not otherwise indicated, the term "about" relates to the indicated value with the commonly accepted technical precision in the relevant field, preferably relates to the indicated value $\pm$ 20%, more preferably $\pm$ 10%, most preferably $\pm$ 5%.

The invention will now be further explained with reference to specific features and steps to be performed.

*A method of characterising the mode of action of a test compound*

The invention provides a method of characterising a mode of action.

In general terms, a mode of action describes a functional, anatomical or molecular mechanism effected from the exposure of a living organism to a substance. In comparison, a mechanism of action describes such changes at the molecular level. A mode of action is important in classifying chemicals as it represents an intermediate level of complexity in between molecular mechanisms and physiological outcomes, especially when the exact molecular target has not yet been elucidated or is subject to debate.

In relation to the present invention, the mode of action can be characterized according to alterations in the isotopolomic profile of a living system exposed to the test compound in comparison to a living system not exposed to the test compound.

Such information is important in the development of, for example, pesticides, since new modes of action can mitigate the effect of pesticide resistance within the target organisms.

*Performing chromatograph and mass spectrometry analysis*

The term "chromatography coupled mass spectrometry" as used herein relates to mass spectrometry which is coupled to a prior chromatographic separation of the metabolites comprised by the samples to be investigated.

Chromatography is a laboratory technique for the separation of a mixture. The mixture is dissolved in a fluid called the mobile phase, which carries it through a structure holding another material called the stationary phase. The various constituents of the mixture travel at different speeds, causing them to separate. The separation is based on differential partitioning between the mobile and stationary phases. Subtle differences in a compound's partition coefficient result in differential retention on the stationary phase and thus affect the separation.

Suitable techniques for separation to be used preferably in accordance with the present invention, therefore, include all chromatographic and/or electrophoretic separation techniques such as liquid chromatography (LC), high performance liquid chromatography (HPLC), ultra performance liquid chromatography (UPLC), gas chromatography (GC), thin layer chromatography, size exclusion, affinity chromatography and capillary electrophoresis (CE). Most preferably, GC, LC, UPLC and/or HPLC are chromatographic techniques to be envisaged by the method of the present invention. Suitable devices for such determination of analyte(s) are well known in the art.

Following the chromatography stage, the metabolites are then analyzed by mass spectrometry.

Mass spectrometry (MS) is an analytical technique that ionizes chemical species and sorts the ions based on their mass to charge ratio (m/z) and detects the ion current intensity or ion count related to this specific m/z. In one example, MS gathers ion counts or measures signals related to the amount of different ions, where the difference of those ions in based on their different m/z. Sorting by m/z can, for example, be done by electrical and/or magnetic fields. In another example of MS function, this information can be gathered by measuring voltages or currents, induced by moving ions, where the movement is caused by electrical and/or magnetic fields. Mass spectrometry is used in many different fields and is applied to pure samples as well as complex mixtures.

A mass spectrum is a plot of the ion signal as a function of the m/z, where the ion signal is a numeric value, which refers to the amount of detected ions related to the corresponding m/z. These spectra are used to determine the elemental and/or isotopic signature of a sample, the masses of particles and of molecules, and to elucidate the chemical structures of molecules, such as peptides and other chemical compounds, as well as the relative amount of different chemical compounds within a sample.

In a typical MS procedure, a sample, which may be solid, liquid, or gas, is ionized, for example by proton transfer or bombarding it with electrons. This may cause some of the sample's molecules to be converted into charged ions, termed "full scan ions". These full scan ions are then separated according to their m/z, typically by accelerating them and subjecting them to an electric and/or magnetic field: full scan ions of the same m/z will undergo the same amount of deflection. The full scan ions are detected by a mechanism capable of detecting charged particles, for example an appliance including an electron multiplier. Results are displayed as spectra of the relative abundance of detected full scan ions as a function of the m/z. Hence mass spectrometry is used to assign one or a group of specific m/z of an ion or ions to a specific metabolite or a mixture of metabolites in the analyzed sample, due to the ionization process.

Mass spectrometry as used herein encompasses all techniques which allow for the determination of the molecular weight (i.e. the mass) or a mass variable corresponding to a compound to be determined in accordance with the present invention. Preferably, mass spectrometry as used herein relates to GC-MS, LC-MS (where LC can be different types of liquid chromatography, such as HPLC or UPLC), direct infusion mass spectrometry, FT-ICR-MS, CE-MS, HPLC-MS. How to apply these techniques is well known to the person skilled in the art. Moreover, suitable devices are commercially available. More preferably, mass spectrometry as used herein relates to LC-MS and/or GC-MS.

*Calculating the mass isotopomer distribution for metabolites from the mass spectra data*

As described above, the method of the invention provides mass spectral data for the metabolities extracted from the biological samples.

The mass spectral data comprises the mass, intensity and retention times of the detected metabolites. This mass spectral data subsequently undergoes a series of data processing steps to produce the mass isotopomer distribution for all the detected metabolites.

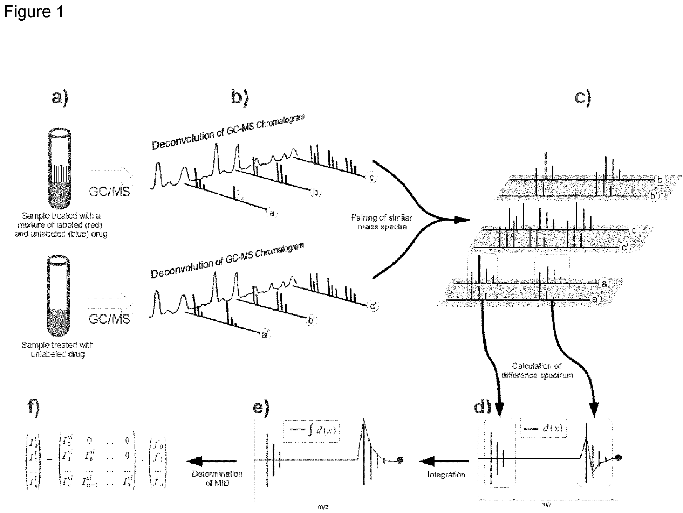

As way of an example, the data processing stages performed are now described in further detail. A schematic diagram of the data processing stages is also provided in Figure 1.

1. Calculating the retention indices and deconvolution of the mass spectral data

An embodiment of the invention is wherein the mass isotopomer distribution generated is determined by: deconvolution of the mass spectral data; pairing the deconvoluted mass spectra data and calculating the retention indices across samples and identifying metabolites according to isotope incorporation; identification of those metabolites based on similarity of spectra, retention time and/or retention index by comparison to a reference library; calculation of the mass isotopomer distribution of the metabolites.

The data generated from the GC-MS analysis of the biological samples are exported from the mass spectrometry apparatus typically in a netCDF file format. The data set for the metabolites comprises multiple variables, including the masses, intensities and retention times of each detected metabolite.

The netCDF file for each sample is imported into an appropriate software to perform the subsequent data analysis steps.

Appropriate software programs to perform this data analysis steps include, for example, MetaboliteDetector software (MetaboliteDetector - Deconvolution and Analysis of GC/MS Data; Version 3.2; http://metabolitedetector.tu-bs. de), or

Xcalibur 4.0 (https://www.thermofisher.com/order/catalog/product/OPTON-30487), or AMDIS 2.73 (https://chemdata. nist.gov/dokuwiki/doku.php?id=chemdata:amdis) or AnalyzerPro 5.5 (https://www.spectralworks.com/analyzerpro. html), or Mzmine 2 (http://mzmine.github.io/)). Other suitable software packages will be well known to the person skilled in the art.

As way of example, we describe the analysis performed by the MetaboliteDetector software (MetaboliteDetector - Deconvolution and Analysis of GC/MS Data; Version 3.2; http://metabolitedetector.tu-bs.de). The software determines the retention index for each detected metabolite, which are retention times expressed in a system independent manner. This can be achieved by using a reference sample, for example, an n-alkane mix, which is also analyzed on the same day and using the same GC-MS apparatus as that which generated the mass spectral data derived from the biological samples. The chromatogram derived from the reference sample is subsequently used as a reference chromatogram for retention index calculation by the MetaboliteDetector software.

The MetaboliteDetector software also performs an ion chromatographic deconvolution on the mass spectral data. This is represented as step b) in Figure 1. This assists in the subsequent data analysis since co-elution and in-source fragmentation cause the resulting raw mass spectra to consist of mass peaks from all of the co-eluting metabolites. To identify and extract the quantitative information of the corresponding metabolites, the spectrum for each single metabolite has to be constructed based on the composite spectra. This spectrum construction step is called *deconvolution* in GC-MS data processing. Deconvolution is the process of computationally separating co-eluting metabolies from the chromatography

Deconvolution is a routine calculation during the analysis of mass spectral data.

2, 3. Pairing the deconvoluted mass spectra data across samples and identifying metabolites according to isotope incorporation

The deconvoluted mass spectral data of metabolites derived from different biological samples are then paired according to the retention index values and spectrum similarity.

Appropriate software programs to perform this data processing step include, for example, MIA (Mass Isotopolome Analyzer; Version 1.0; https://mia.bioinfo.nat.tubs.de/) or NTFD (http://ntfd.bioinfo.nat.tu-bs.de), or

X13CMS (http://pattilab.wustl.edu/software/x13cms/x13cms.php), or
IROA 345 (http://www.iroatech.com).

As way of example, we describe the analysis performed by the NTFD algorithm as implemented in the MIA (Mass Isotopolome Analyzer; Version 1.0; https://mia.bioinfo.nat.tu-bs.de/) software package. This is represented by step c) in Figure 1.

Following this process, the data is analyzed to identify mass spectra data sets derived from relevant metabolites, i.e. metabolites from biological samples which have incorporated an isotope. This analysis is based on identifying metabolites which have an isotopic difference between the different biological samples.

The NTFD is able to identify such metabolites on the basis of certain criteria, including: relative intensities from unlabeled fragments have same value as in the spectrum measured for unlabeled compound alone; relative signal of first peak of a fragment of mixture labeled and unlabeled is always lower than the signal of corresponding peak of the unlabled molecule; the sum of relative intensities of a fragment is independent of the amount of isotopic enrichment.

The NTFD also performs a series of additional calculations to determine metabolites. The NTFD calculates a difference spectrum $d(x)$ for each detected compound as follows: as shown in step d) of Figure 1 the MID for unlabeled is put in the positive direction (above the axis), MID for labeled is put in the negative direction (below the axis). When calculating the difference of both, it becomes clear that labeled ion fragments form a shape similar to first derivative of peak function, while in contrast unlabeled ion fragments are eliminated (left) = rule 1. This difference spectrum is integrated over the whole spectrum to obtain the integral of $d(x)$ (step e in Figure 1). Peaks of this function are labeled fragments. Hence this peak detection delivers you with isotopically labeled metabolites and thus relevant metabolites.

<u>4. Identification of metabolites based on similarity of spectra retention time and/or retention index by comparison to a reference library</u>

Once the mass spectra data of labeled metabolites has been detected, compound identification is performed using libraries of spectra data; see, for example the Golm Metabolome Database (http://gmd.mpimp-golm.mpg.de/). This analysis is based on the similarity of spectra, retention time and/or retention index, depending on the data available in the selected metabolite library. Such data processing steps are routine in the analysis of mass spectra data.

The previous data processing steps have thus provided a collection of mass spectra data of relevant metabolites. This data needs to be prepared as a metabolite library for the MetaboliteDetector software (described above). The spectra need to be exported as a metabolite library. The data comprises the unlabeled spectra of all labeled metabolites containing the metabolite spectra from the unlabeled samples, ions, the identified name, retention time and retention index. The metabolite library export can be performed either with the MIA or NTFD software.

5. Calculation of mass isotopomer distributions for all detected compounds

The metabolite library from the previous step is then used to calculate the mass isotopomer distribution (MID) for all previously detected metabolites that are stored in the metabolite library. The MID is calculated using a set of linear equations to elucidate the composition of mixtures of isotopomers for each detected labeled fragment (step f of Figure 1). An example of such an algorithm is presented in Figure 1. This analysis can be performed using, for example, the MetaboliteDetectors MID wizard (other software packages have equivalent wizards or add-ons to calculate MIDs). The files containing the labeled spectra are used for a targeted search with the previously created metabolite library.

An important advantage of using NTFD analysis is that the MIDs of unknown metabolites as well as known metabolities can be determined. Hence the effect of test compounds on unknown and previously unidentified biochemical pathways can be measured using the method of the invention.

<u>6. Correction of MID values for naturally occurring isotope abundances</u>

This data processing step is standard in mass spectrometry analysis and would be clearly understood by the skilled person. It is automatically performed in MetaboliteDetector, NTFD or MIA when calculating the MID.

The MID are exported in a machine-readable format.

Hence the data processing steps provided above results in the calculation of the MID for the metabolites from the mass spectra data, as stated in step d) of the method of the invention.

*Identifying metabolites that have a significant different isotopomer distribution between the biological samples*

Step d) of the method of the invention provides MID of the analysed metabolites. The method of the invention includes step e) of identifying metabolites that have a significant different isotopomer distribution

It can be understood by the skilled person that those metabolites having significantly different MID values between the biological samples can be readily identified by an examination of the data for the same metabolites from different biological samples.

This can be performed using a number of different data processing steps. By way of example, this stage of the method of the invention can be performed as set out below.

*Variability analysis*

The variability between the selected common ions is the difference between the unlabeled mass isotopomer M0 of treatment and control.

*Sorting*

Under the assumption that the $^{13}$C tracer (or any other isotope used in the method of the invention) distributes through the organism starting from the entry point with a decreasing fractional contribution towards more downstream metabolites, the detected metabolites can be sorted based on the total amount of labeling. The fractional contribution (FC) is the amount of labeling of the metabolite related to the number of carbon atoms M (Eq 1), where $m_i$ is the ith mass isotopomer.

Equation 1

$$FC = \frac{\sum_{i=0}^{M} m_i \cdot i}{M} \quad (1)$$

A higher FC indicates a closer proximity to the tracer than a metabolite with a lower FC.

*MID distances*

The MID based distance calculation uses the Needleman-Wunsch algorithm for gap filling for unequal number of isotopomers and a distance measure (e.g. canberra distance, manhattan distance, etc.) to determine the distance between all labeled metabolites.

Hence using the analysis provided above, the skilled person would be able to identify those metabolites which have significantly different isotopomer distribution when the living system was exposed to the test compound to the isotopomer distribution when the living system was not exposed to the test compound, as required by step e) of the method of the invention.

*Characterizing the mode of action of the test compound*

An embodiment of the method of the invention is wherein the mode of action of the test compound is identified according to the metabolites identified from step e).

Based on fractional contribution and MID distances, biochemical pathways can be constructed without *a priori* biochemical knowledge. Metabolites with similar fractional contribution and MIDs are considered to belong to the same pathway and are therefor put closely together in the pathway. This pathway construction is done for the control and for the treatment. Those connections between metabolites that are present in the control and are not anymore on the treatment suggest enzymatic activities that are blocked by eg a pesticidal working mechanism.

This analysis is combined with knowledge about the detected metabolites. Hence if it is known that metabolites 'x' and 'y' are part of biochemical pathway 'z', then the mode of action of the test compound can be assigned to that specific pathway. Here the identification of the detected metabolites is important for the extrapolation of the enzymatic reactions.

*Isotopic differences between biological samples*

The present invention is directed to methods of characterizing the mode of action of a test compounds in living systems by measuring differences in isotopomer distribution in metabolites from biological samples derived from a living system exposed to the test compound in comparison to a living system not exposed to the test compound.

The method uses biological samples derived from living systems having at least one isotopic difference.

To generate biological samples that can be used in the method of the invention, at least one isotope-labeled substrate molecule is administered to a living system (for example one or more cells, tissues or organisms) for a period of time sufficient to be incorporated into metabolic pathways. In parallel a further living system is exposed to the same substrate molecule which does not have the isotope label.

The living systems exposed to the isotopically-labeled substrate molecule or non-isotopically labeled substrate molecule thus provides the biologically samples having least one isotopic difference between the samples, as used in the method of the invention.

The method of the invention uses at least four biological samples derived from living systems.

The first and second biological samples are derived from living systems which have been exposed to the test compound. There is an isotopic difference between the first and second biological samples, as described above.

The third and fourth biological samples are derived from living systems which have not been exposed to the test compound. There is an isotopic difference between the third and fourth biological samples, as described above.

As will be appreciated by the skilled person, the four different biological samples can have any nomenclature as appropriate.

As will also be appreciated, the method of the invention can use more than four different biological samples. For example, the method could be part of a screening system in which multiple living systems are exposed to varying amount of the test compound, or to different test compounds.

It is preferred that, within a use of the method of the invention, the biological samples are derived from living systems which are the same but which have been exposed to differing isotopic and test compound regimes, as described herein.

In one embodiment, the isotope-labeled substrate molecules are labeled with one or more stable isotopes (i.e., non-radioactive isotope). In another embodiment, the isotope-labeled substrate molecule is labeled with one or more radioactive isotopes.

In yet another embodiment, both stable and radioactive isotopes are used to label one or more isotope-labeled substrate molecules.

Isotope labels that can be used in accordance with the methods of the present invention include, but are not limited to $^2H_2O$, $H_2$ $^{18}O$, $^2H$-glucose, $^2H$-labeled amino acids, $^2H$-labeled organic molecules, $^{13}C$-labeled organic molecules, $^{13}$glycerol, $^{13}CO_2$, $^{15}N$-labeled organic molecules, $^3H_2O$, $^3H$-labeled glucose, $^3H$-labeled amino acids, $^3H$-labeled organic molecules.

"Isotope labeled substrate" includes any isotope-labeled precursor molecule that can be incorporated into a living system.

Compositions comprising carbohydrates may include monosaccharides, polysaccharides, or other compounds attached to monosaccharides or polysaccharides.

Isotope labels may be incorporated into carbohydrates or carbohydrate derivatives by biochemical pathways known in the art. These include monosaccharides (including, but not limited to, glucose and galactose), amino sugars (such as N-Acetyl Galactosamine), polysaccharides (such as glycogen), glycoproteins (such as sialic acid) glycolipids (such as galactocerebrosides), and glycosaminoglycans (such as hyaluronic acid, chondroitin-sulfate, and heparan-sulfate).

$^2H$-labeled sugars may be administered to a living system as monosaccharides or as polymers comprising monosaccharide residues. Labeled monosaccharides may be readily obtained commercially.

Relatively low quantities of compounds comprising $^2H$-labeled sugars need be used. Quantities may be on the order of milligrams, 101mg, 102mg, 103mg, 104mg, 105mg, or 106mg. $^2H$-labeled sugar enrichment may be maintained for weeks or months in humans and in animals without any evidence of toxicity. The low cost of commercially available labeled monosaccharides, and low quantity that need to be administered, allow maintenance of enrichments at low expense.

In one embodiment, the labeled sugar is glucose.

In another embodiment the "isotope labeled substrate" is $CO_2$.

In another embodiment the "isotope labeled substrate" is glycerol.

*Living systems*

The method of the invention uses biological samples which are derived from living system.

The living system may be an intact organism or a tissue or cell line derived from a organism.

The living system may be a plant, fungus, bacteria, invertebrate or vertebrate including mammals.

Preferably, said living system is an animal. Said living system may be a mammal, for example a rodent, preferably a mouse rat or rabbit. The living system can also be a cell line from a mammal, for example a human cell line.

Preferably, said living system is an invertebrate, for example a nematode, preferably Caenorhabditis elegans. The living system can also be a cell line from an invertebrate.

Preferably, said living system is an arthropod. Preferably, said living system is an insect. Preferably, said living system is a fly. Preferably, said living system is Drosophila melanogaster, Drosophila simulans, or Drosophila virilis.

Preferably, said living system is a plant, or a cell line derived from a plant.

As used herein, the term "plant" relates to a whole plant, a plant part, a plant organ, a plant tissue, or a plant cell. Thus, the term includes, preferably, seeds, shoots, stems, leaves, roots (including tubers), and flowers. Preferably, the term "plant" relates to a member of the clade Archaeplastida.

Plants that are particularly useful in the methods of the invention include all plants which belong to the superfamily Viridiplantae, preferably Tracheophyta, more preferably Spermatophytina, most preferably monocotyledonous and dicotyledonous plants including fodder or forage legumes, ornamental plants, food crops, trees or shrubs selected from the list comprising Acer spp., Actinidia spp., Abelmoschus spp., Agave sisalana, Agropyron spp., Agrostis stolonifera, Allium spp., Amaranthus spp., Ammophila arenaria, Ananas comosus, Annona spp., Apium graveolens, Arachis spp, Artocarpus spp., Arabidopsis thaliana, Asparagus officinalis, Avena spp. (e.g. Avena sativa, Avena fatua, Avena byzantina, Avena fatua var. sativa, Avena hybrida), Averrhoa carambola, Bambusa sp., Benincasa hispida, Bertholletia excelsea, Beta vulgaris, Brassica spp. (e.g. Brassica napus, Brassica rapa ssp. [canola, oilseed rape, turnip rape]), Cadaba farinosa, Camellia sinensis, Canna indica, Cannabis sativa, Capsicum spp., Carex elata, Carica papaya, Carissa macrocarpa, Carya spp., Carthamus tinctorius, Castanea spp., Ceiba pentandra, Cichorium endivia, Cinnamomum spp., Citrullus lanatus, Citrus spp., Cocos spp., Coffea spp., Colocasia esculenta, Cola spp., Corchorus sp., Coriandrum sativum, Corylus spp., Crataegus spp., Crocus sativus, Cucurbita spp., Cucumis spp., Cynara spp., Daucus carota, Desmodium spp., Dimocarpus longan, Dioscorea spp., Diospyros spp., Echinochloa spp., Elaeis (e.g. Elaeis guineensis, Elaeis oleifera), Eleusine coracana, Eragrostis tef, Erianthus sp., Eriobotrya japonica, Eucalyptus sp., Eugenia uniflora, Fagopyrum spp., Fagus spp., Festuca arundinacea, Ficus carica, Fortunella spp., Fragaria spp., Ginkgo biloba, Glycine spp. (e.g. Glycine max, Soja hispida or Soja max), Gossypium hirsutum, Helianthus spp. (e.g. Helianthus annuus), Hemerocallis fulva, Hibiscus spp., Hordeum spp. (e.g. Hordeum vulgare), Ipomoea batatas, Juglans spp., Lactuca sativa, Lathyrus spp., Lemna paucicostata, Lens culinaris, Linum usitatissimum, Litchi chinensis, Lotus spp., Luffa acutangula, Lupinus spp., Luzula sylvatica, Lycopersicon spp. (e.g. Lycopersicon esculentum, Lycopersicon lycopersicum, Lycopersicon pyriforme), Macrotyloma spp., Malus spp., Malpighia emarginata, Mammea americana, Mangifera indica, Manihot spp., Manilkara zapota, Medicago sativa, Melilotus spp., Mentha spp., Miscanthus sinensis, Momordica spp., Morus nigra, Musa spp., Nicotiana spp., Nicotiana tabacum, Olea spp., Opuntia spp., Ornithopus spp., Oryza spp. (e.g. Oryza sativa, Oryza latifolia), Panicum miliaceum, Panicum virgatum, Passiflora edulis, Pastinaca sativa, Pennisetum sp., Persea spp., Petroselinum crispum, Phalaris arundinacea, Phaseolus spp., Phleum pratense, Phoenix spp., Phragmites australis, Physalis spp., Pinus spp., Pistacia vera, Pisum spp., Poa spp., Populus spp., Prosopis spp., Prunus spp., Psidium spp., Punica granatum, Pyrus communis, Quercus spp., Raphanus sativus, Rheum rhabarbarum, Ribes spp., Ricinus communis, Rubus spp., Saccharum spp., Salix sp., Sambucus spp., Secale cereale, Sesamum spp., Sinapis sp., Solanum spp. (e.g. Solanum tuberosum, Solanum integrifolium or Solanum lycopersicum), Sorghum bicolor, Spinacia spp., Syzygium spp., Tagetes spp., Tamarindus indica, Theobroma cacao, Trifolium spp., Tripsacum dactyloides, Triticosecale rimpaui, Triticum spp. (e.g. Triticum aestivum, Triticum durum, Triticum turgidum, Triticum hybernum, Triticum macha, Triticum sativum, Triticum monococcum or Triticum vulgare), Tropaeolum minus, Tropaeolum majus, Vaccinium spp., Vicia spp., Vigna spp., Viola odorata, Vitis spp., Zea mays, Zizania palustris, Ziziphus spp., amongst others.

The living system may be a pest of plants, animals or humans. Examples of pest species include aphids, thrips, locust, whitefly, black fly, leafhoppers, mosquitoes, nematodes, wasps, termites, rice hoppers, rice bugs, mealy bugs, white grubs, Colorado potato beetle, flea beetle, lice, mites, ants, fleas, tics, wireworms, ground beetles, leaf miners, butterflies, moths, weevils, spiders, spider mites, lacewings, gnats, midges, flies, bees, plant hoppers, biting insects, sucking insects and lawn pests. The term "pest species" includes parasites.

Alternatively the living system may be a species beneficial to a crop species (for example by acting as a predator, parasite or competitor to a pest species or by acting to improve soil condition or as a pollinator). Examples of beneficial species include earthworms, butterflies, moths and bees.

Alternatively the living system may be a fungus. "Fungus" includes a wide variety of nucleated spore-bearing organisms that are devoid of chlorophyll. Examples of fungi include yeasts, molds, mildews, rusts, and mushrooms. In particular, the fungus may be *Magnaporthe oryzae, Botrytis cinereal, Puccinia* spp., *Fusarium graminearum, Fusarium oxysporum, Blumeria graminis, Mycosphaerella graminicola, Colletotrichum* spp., *Ustilago maydis or Melampsora lini., Pyricularia oryzae.*

Alternatively the living system may be a bacteria. "Bacteria" includes a wide variety of single-celled prokaryotic organisms, and includes the following classifications and genera: Bacillus, Pseudomonas, Erwinia, Serratia, Klebsiella, Xanthomonas, Streptomyces, Rhizobium, Rhodopseudomonas, Methylius, Agrobacterium, Acetobacter, Lactobacillus, Arthrobacter, Azotobacter, Leuconostoc, Alcaligenes, Pectobacterium, Pantoea, Acidovorax, Clavibacter, Xylella, Spiroplasma and Phytoplasma.

*Metabolites*

The term "metabolite", as used herein, relates to at least one molecule of a specific metabolite up to a plurality of molecules of the said specific metabolite. It is to be understood further that a group of metabolites means a plurality of chemically different molecules wherein for each metabolite at least one molecule up to a plurality of molecules may be present. A metabolite in accordance with the present invention encompasses all classes of organic or inorganic chemical compounds including those being comprised by biological material such as animals or plants. Preferably, a metabolite has a molecular weight of from 25 Da (Dalton) to 300,000 Da, more preferably of from 30 Da to 30,000 Da, most preferably of from 50 Da to 1500 Da. Preferably a metabolite has a molecular weight of less than 30,000 Da, less than 20,000 Da, less than 15,000 Da, less than 10,000 Da, less than 8,000 Da, less than 7,000 Da, less than 6,000 Da, less than 5,000 Da, less than 4,000 Da, less than 3,000 Da, less than 2,000 Da, less than 1,500 Da, less than 1,000 Da, less than 500 Da, less than 300 Da, less than 200 Da, or less than 100 Da. Preferably, a metabolite has, however, a molecular weight of at least 50 Da.

Preferably, the metabolite is a biological macromolecule, e.g. preferably, DNA, RNA, protein, or a fragment thereof, e.g., preferably a fragment produced by processing of sample material. More preferably, in case a plurality of metabolites is envisaged, said plurality of metabolites is representing a metabolome, i.e. the collection of metabolites being comprised by an organism, an organ, a tissue, a body fluid, a cell or a part of a cell at a specific time and under specific conditions.

More preferably, the metabolite in accordance with the present invention is a small molecule compound, such as a substrate for an enzyme of a metabolic pathway, an intermediate of such a pathway or a product obtained by a metabolic pathway. Metabolic pathways are well known in the art and may vary between species. Preferably, said pathways include at least citric acid cycle, respiratory chain, photo respiratory chain, glycolysis (Embden-Meyerhof-Parnas (EMP) pathway), gluconeogenesis, hexose monophosphate pathway, starch metabolism, oxidative and non oxidative pentose phosphate pathway (Calvin-Benson (CB) cycle, glyoxylate metabolism, production and β-oxidation of fatty acids, urea cycle, amino acid biosynthesis pathways, protein degradation pathways such as proteasomal degradation, amino acid degrading pathways, biosynthesis or degradation of lipids, polyketides (including e.g. flavonoids and isoflavonoids), isoprenoids (including eg. terpenes, sterols, steroids, carotenoids, xanthophylls), carbohydrates, phenylpropanoids and derivatives, alcaloids, benzenoids, indoles, indole-sulfur compounds, porphyrines, anthocyans, hormones, vitamins, cofactors such as prosthetic groups or electron carriers, lignin, glucosinolates, purines, pyrimidines, nucleosides, nucleotides and related molecules such as tRNAs, microRNAs (miRNA) or mRNAs. Accordingly, small molecule compound metabolites are preferably composed of the following classes of compounds: alcohols, alkanes, alkenes, alkines, aromatic compounds, ketones, aldehydes, carboxylic acids, esters, amines, imines, amides, cyanides, amino acids, peptides, thiols, thioesters, phosphate esters, sulfate esters, thioethers, sulfoxides, ethers, or combinations or derivatives of the aforementioned compounds. The small molecules among the metabolites may be primary metabolites which are required for normal cellular function, organ function or animal or plant growth, development or health. Moreover, small molecule metabolites further comprise secondary metabolites having essential ecological function, e.g. metabolites which allow an organism to adapt to its environment. Furthermore, metabolites are not limited to said primary and secondary metabolites and further encompass artificial small molecule compounds. Said artificial small molecule compounds are derived from exogenously provided small molecules which are administered or taken up by an organism but are not primary or secondary metabolites as defined above, including, preferably, drugs, herbicides, fungicides, and insecticides. Moreover, artificial small molecule compounds may be metabolic products of compounds taken up, and preferably metabolized, by metabolic pathways of an organism. Moreover, small molecule compounds preferably include compounds produced by organisms living in, on or in close vicinity to an organism, more preferably by an infectious agent as specified elsewhere herein, by a parasitic and/or by a symbiotic organism.

*Test compounds*

The invention provides a method of characterising the mode of action of a test compound.

The term 'test compound' relates to any molecule for which it is desired to understand its mode of action. It can be a small molecule or a larger biological molecule; for example, a pesticide peptide compound.

Preferably the test compound is a screening lead in a pesticide product development program.

The term "pesticidal", as used herein, refers to the ability of a substance to decrease the rate of growth of a pest, i.e., an undesired organism, or to increase the mortality of a pest.

**Figure legends**

**Figure 1:** Outline for Calculating the mass isotopomer distribution for metabolites from the mass spectra data. (a) Samples to which the method can be applied; (b) Ion chromatographic deconvolution of mass spectral data; (c) deconvoluted mass spectral data of metabolites derived from different biological samples are then paired according to the retention index values and spectrum similarity; (d) paired mass spectra are applied to compute the difference spectrum d(x) for each detected compound. MID for unlabeled is put in the positive direction (above the axis), MID for labeled is put in the negative direction (below the axis). Labeled ion fragments form a shape similar to first derivative of peak function, while in contrast unlabeled ion fragments are eliminated (left); (e) This difference spectrum is integrated over the whole spectrum to obtain the integral of d(x); (f) MID is calculated using a set of linear equations to elucidate the composition of mixtures of isotopomers for each detected labeled fragment.

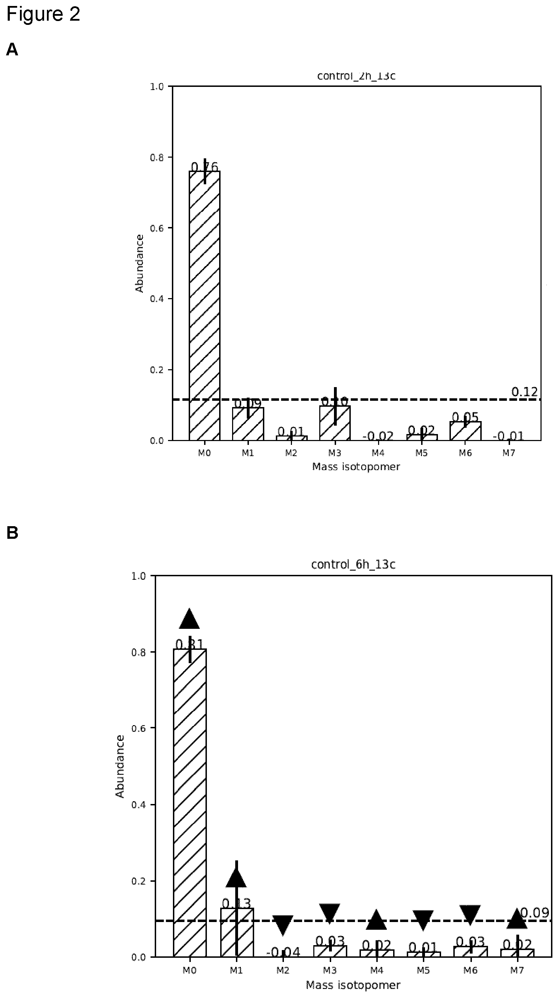

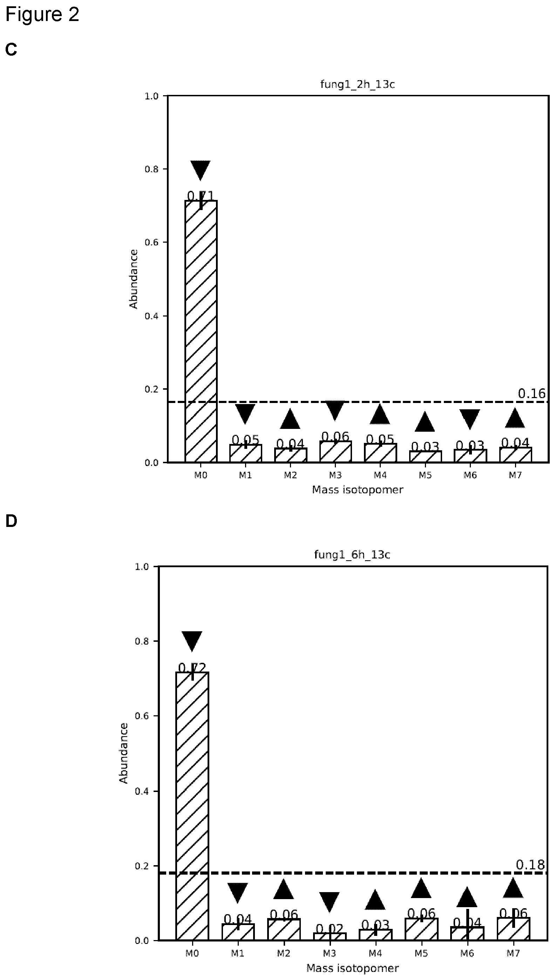

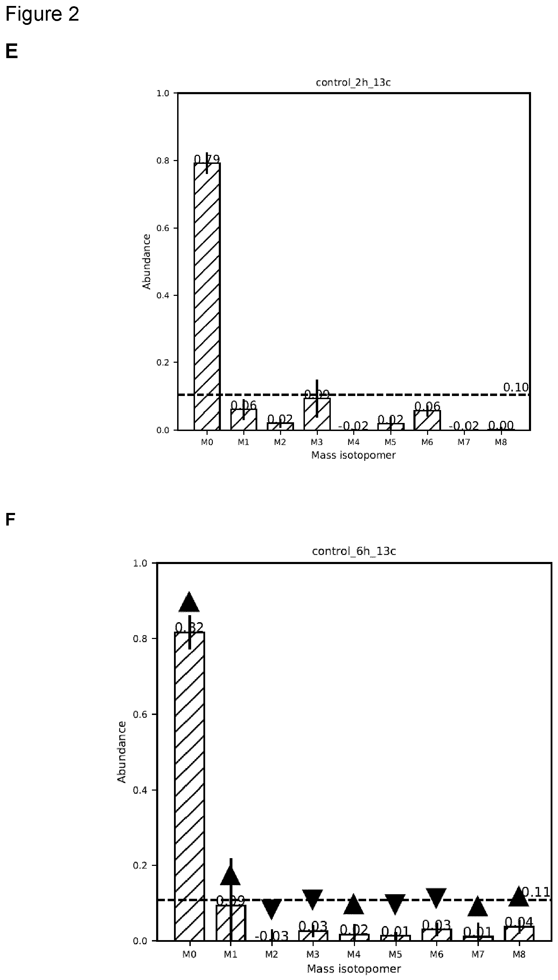

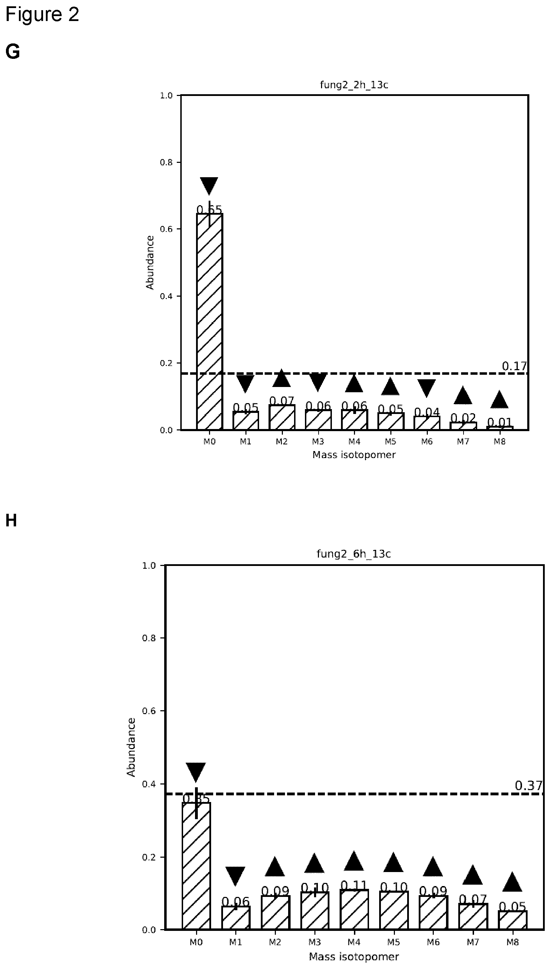

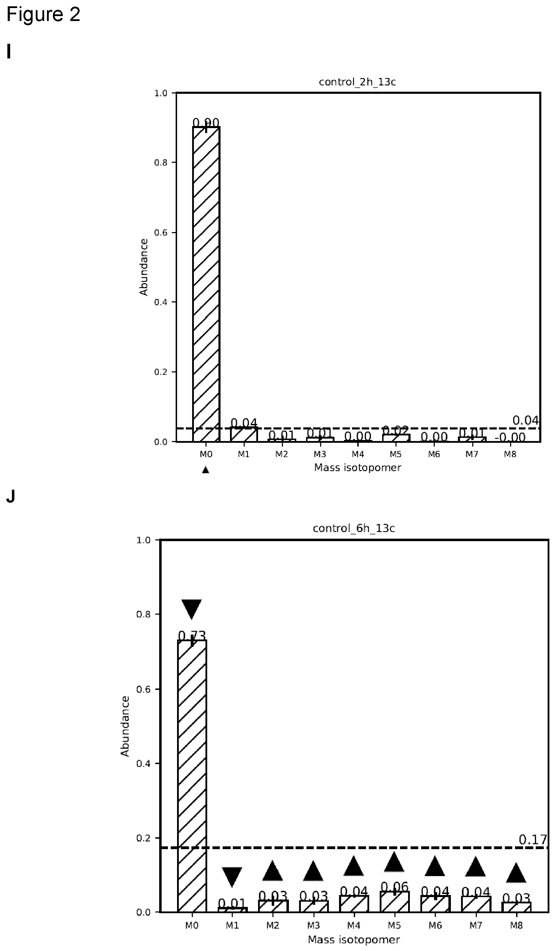

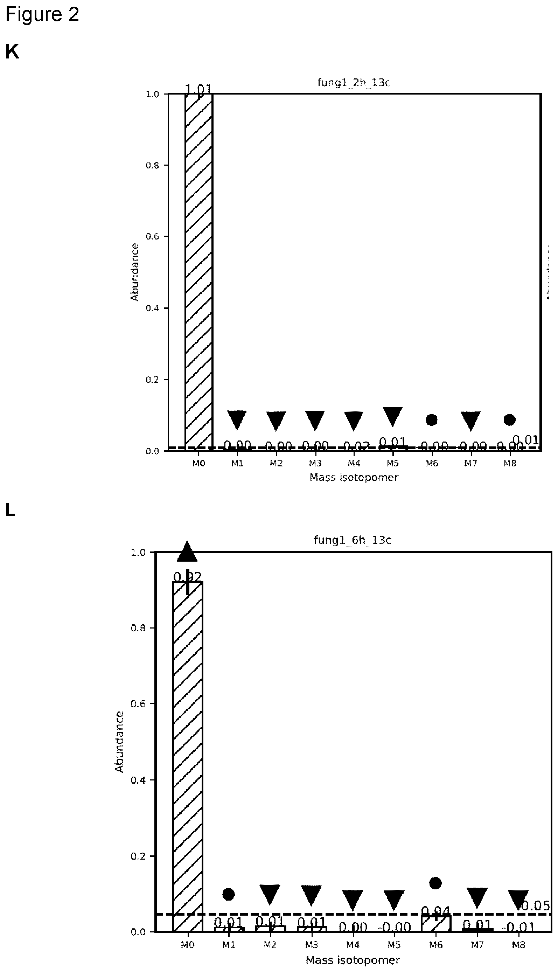

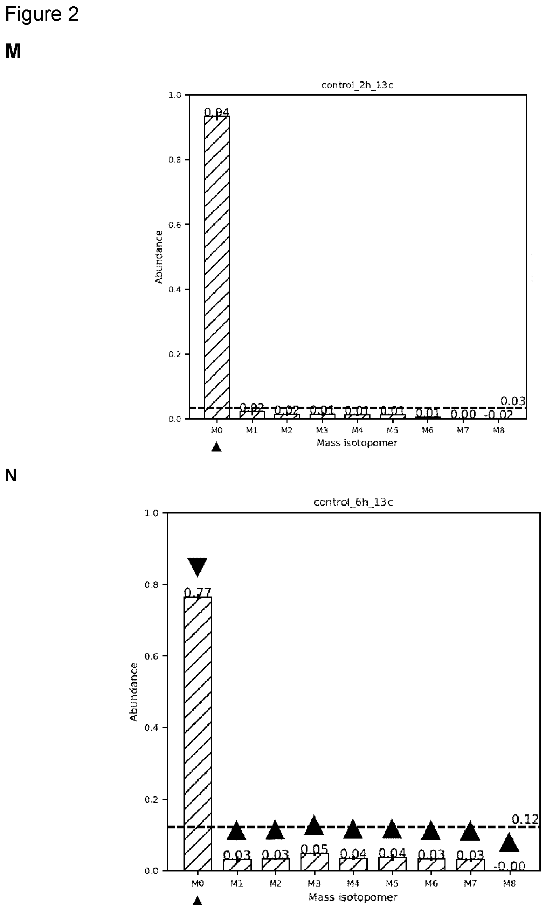

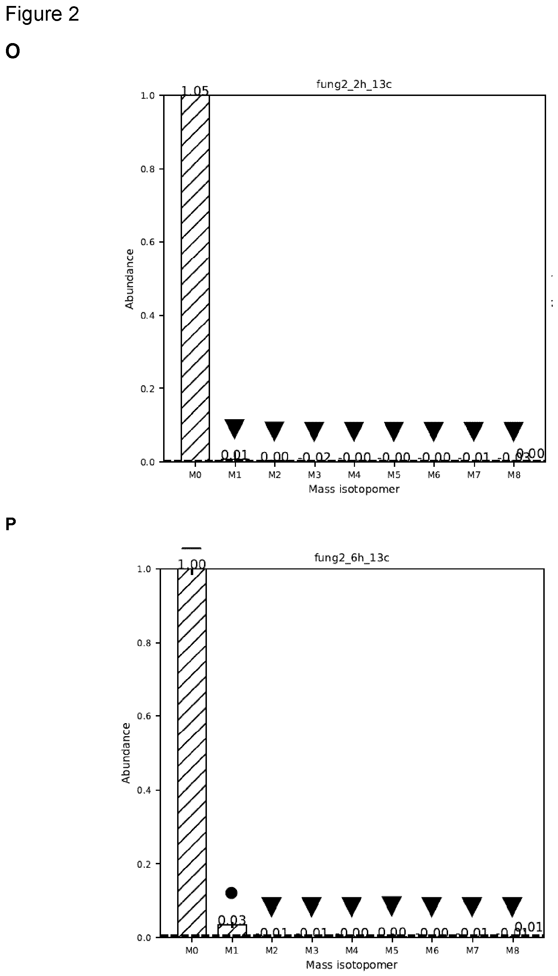

**Figure 2:** Mass isotope distribution (MID) of two unidentified metabolites, RI 3506 and RI 3309 for fungicide one and fungicide two. The 2 h and 6 h graphs differ in incubation time after treatment. Each individual graph shows the mass distribution (M0, M1, M2, ... Mn for n = number of $^{13}$C atoms in the molecule) of the metabolite for that particular combination of treatment and incubation time point. The decrease and increase triangles visualize how the MID has changed in comparison to the respective control. Grey circles represent unchanged MIDs. The dashed line indicates the fractional contribution of the metabolite (fraction of $^{13}$C atoms in the metabolite). (A): RI 3506 MID values for control fungicide one (2 hours); (B): RI 3506 MID values for control fungicide one (6 hours); (C): RI 3506 MID values for fungicide one treatment (2 hours); (D): RI 3506 MID values for fungicide one treatment (6 hours); (E) : RI 3506 MID values for control fungicide two (2 hours); (F): RI 3506 MID values for control fungicide two (6 hours); (G): RI 3506 MID values for fungicide two treatment (2 hours); (H): RI 3506 MID values for fungicide two treatment (6 hours); (I): RI 3309 MID values for control fungicide one (2 hours); (J): RI 3309 MID values for control fungicide one (6 hours); (K): RI 3309 MID values for fungicide one treatment (2 hours); (L): RI 3309 MID values for fungicide one treatment (6 hours); (M) : RI 3309 MID values for control fungicide two (2 hours); (N): RI 3309 MID values for control fungicide two (6 hours); (O): RI 3309 MID values for fungicide two treatment (2 hours); (P): RI 3309 MID values for fungicide two treatment (6 hours).

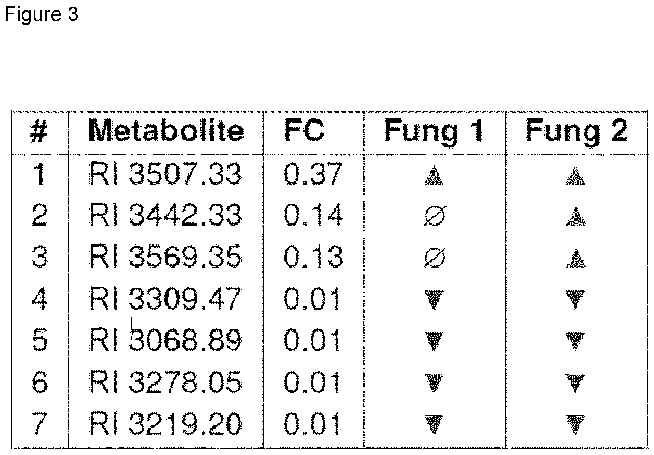

**Figure 3:** The metabolites displaying the largest treatment effects for fungicide one and two are listed. As none of these metabolites could be uniquely identified using the NIST data base, their retention indices (RI) are used as identifiers.

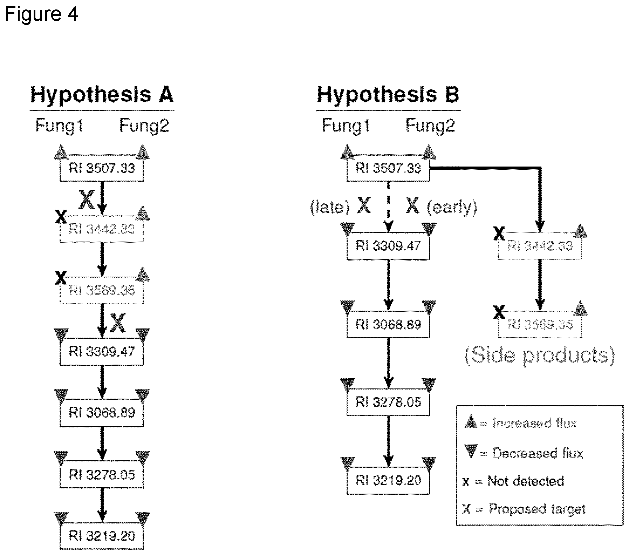

**Figure 4:** Two hypotheses on the differences in mode-of-action between fungicide one and two. The boxed represent (unidentified) metabolites and contain the retention index ID. The connecting arrows indicate one or more enzymatic steps between the metabolites.

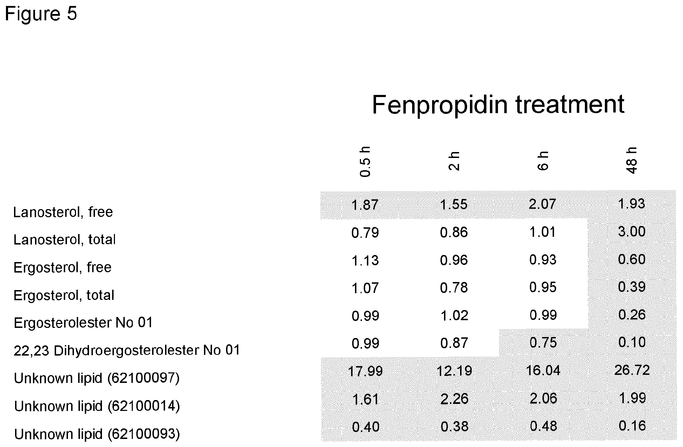

**Figure 5:** Metabolomics results of fenpropidin treatment for samples taken 0.5, 2, 6 and 48 hours after treatment. The values shown represent fold-changes to the control. The grey background indicates significant increases in metabolite concentration.

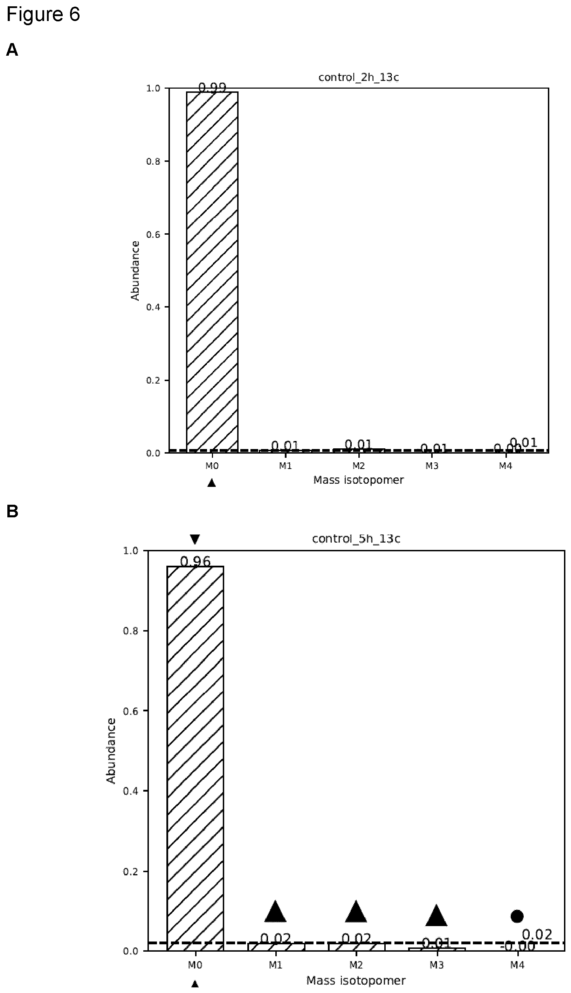

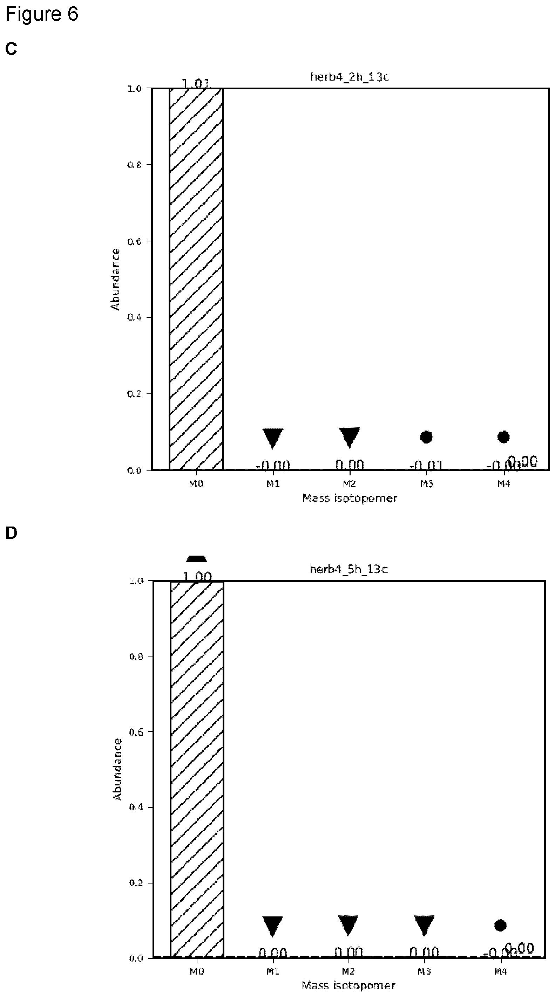

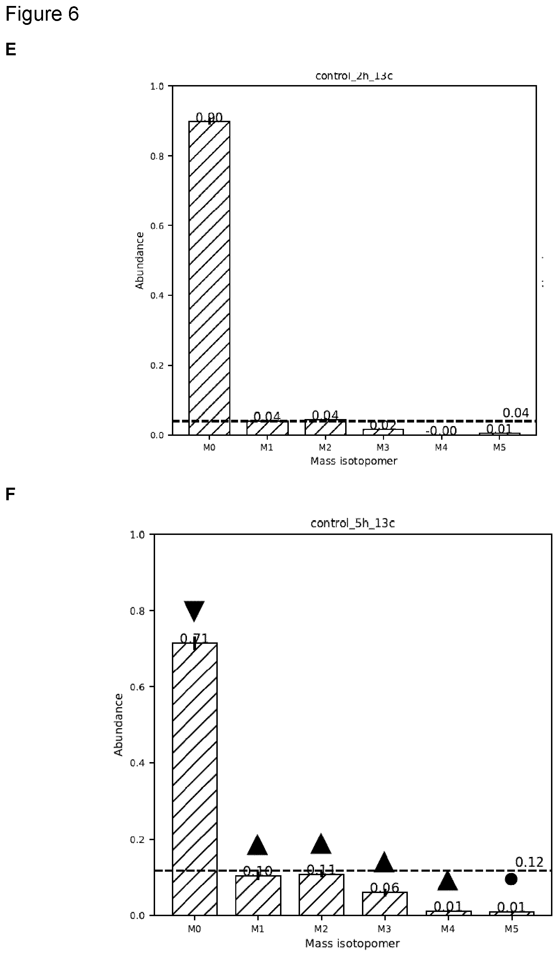

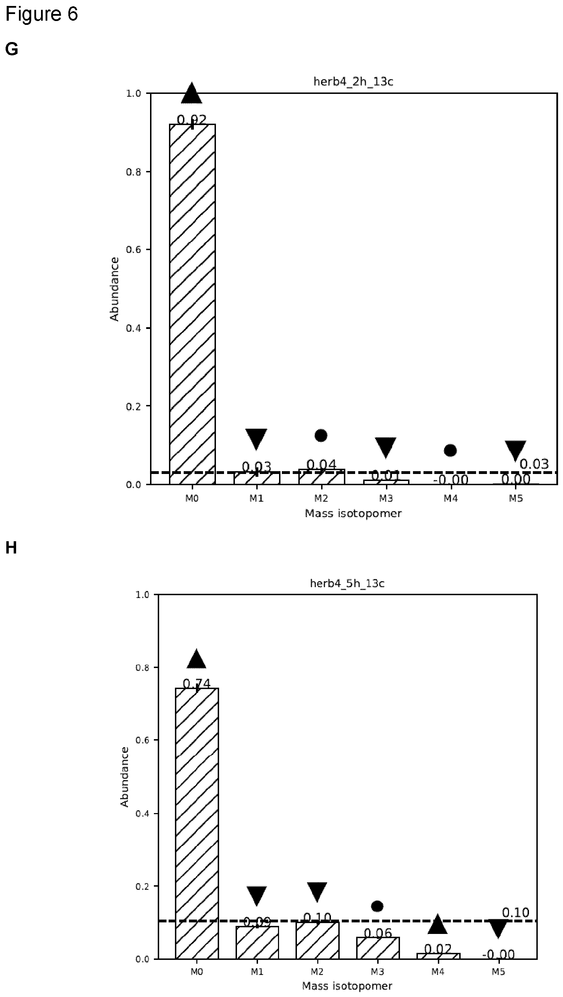

**Figure 6:** Mass isotope distribution (MID) of valine and glutamic acid for herbicide four. Each individual graph shows the mass distribution (M0, M1, M2, ... Mn for n = number of $^{13}$C atoms in the molecule) of the metabolite for that particular combination of treatment and incubation time point. The decrease and increase triangles visualize how the MID has changed in comparison to the respective control. Grey circles represent unchanged MIDs. The dashed line indicates the fractional contribution of the metabolite (fraction of $^{13}$C atoms in the metabolite). (A) Valine MID values for control herbicide four (2 hours); (B): Valine MID values for control herbicide four (5 hours); (C): Valine MID values for herbicide four treatment (2 hours); (D): Valine MID values for herbicide four treatment (5 hours); (E): glutamic acid values for control herbicide four (2 hours); (F): glutamic acid MID values for control herbicide four (5 hours); (G): glutamic acid MID values for herbicide four treatment (2 hours); (H): glutamic acid MID values for herbicide four treatment (5 hours).

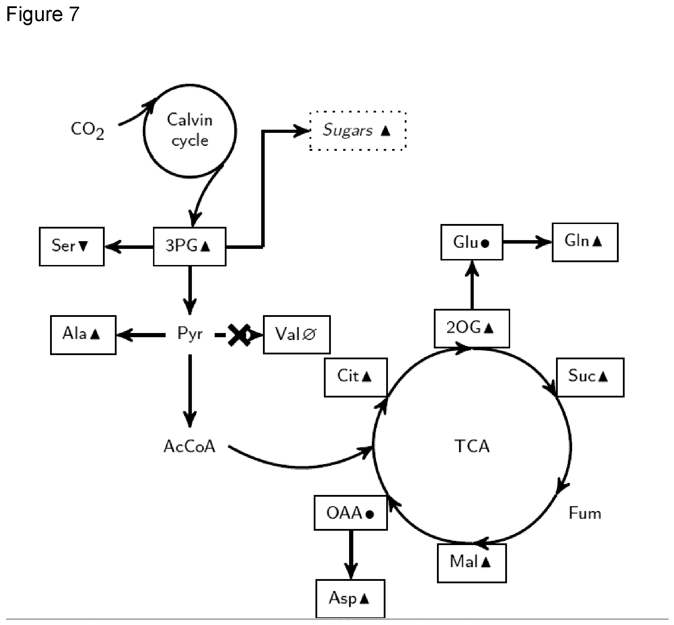

**Figure 7:** Mode-of-action hypothesis for herbicide four. The boxes represent metabolites and contain the annotation or the retention index ID of the metabolites. The connecting arrows indicate one or more enzymatic steps between the metabolites. Whenever an increase in label incorporation is detected, the metabolite box is equipped with an increase triangle. Decreases are represented by a triangle pointing downward. Unchanged label patterns are indicated by a grey circle, whereas the symbol for an empty set is used if no label could be detected. The crosses indicate the mode-of-action hypothesis. Measured metabolites are in black boxes, boxes with a dashed line and grey background represent groups of metabolites and if a metabolite was analyzed in a targeted way, this is represented by a light grey dashed box.

The invention will now be described further by way of the following non limiting examples.

## Example 1: Mode of action identification

**Introduction**

"Screening leads" in pesticide development are compounds with high efficacy and potency that additionally display an interesting mode-of-action during initial screening. Approximately half of these promising compounds have a mode-of-action that is not elucidated easily, and they proceed into the advanced mode-of-action analysis. These latter compounds are especially interesting as potentially new pesticide classes with entirely new modes-of-action are discovered. However, elucidating a new mode-of-action is particularly difficult with only a limited number of methods available in the advanced mode-of-action analysis pipeline.

Metabolite profiling for mode-of-action identification is one approach to determine the mode-of-action of screening leads. However, current metabolic profiling methodologies can only detect major metabolic changes, as interfering secondary effects disguise the primary mode-of-action.

Against this background the present inventors sought to use isotopic tracers (for example $^{13}$C) to distinguish between primary and secondary effects. Surprisingly they determined that this new approach provides an improved methodology identify the mode-of-action for promising compounds. The improved methodology has been termed MIAMI (Mass Isotopolome Analysis for Mode-of-Action Identification).

Advantages of the improved method of the invention include: increase sensitivity and broader scope of application than existing methods, to create a generalized platform for identification of the primary mode-of-action.

Further benefits of MIAMI are:

- The method is untargeted and thus not limited to known metabolites or pathways. A broader analytical scope will allow for the identification of novel modes-of-action and new biomarkers.

- Using isotopic tracers will increase the capability to identify modes-of-action by metabolic profiling, as it has an increased sensitivity, allowing for the identification of primary effects in highly variable biological systems.

- A single combined analytical and computational platform can be used for multiple purposes, e.g. pesticide discover, toxicology assessment and white biotechnology and toxicology.

MIAMI improves existing methods for the identification of mode-of-action. This knowledge can allow more rational optimization of compounds for the on-target and to identify (toxicological) off-targets in pesticide development. Therefor this technology can further speed up research projects and identify possible risks early on.

*Mass isotopolome analysis*

The measurement of the isotopomer distribution (~enrichment) of each metabolite adds an important dimension to metabolic data. The challenge lies in how to extract the knowledge from this big and complex data. Recent advances in systems biology (Weindl et al., (2016) Bioinformatics, vol 32(18) 2875-2876) deliver an intelligent solution by building similarity networks and identifying differential enzyme activities.

For this purpose, separate cultivations are done under control and treatment conditions using an unlabeled and labeled carbon source (for example labeled glucose or labeled $CO_2$). Samples are taken, prepared and measured with gas chromatography/mass spectrometry in SCAN mode. After deconvolution of the mass spectra, NTFD (Hiller K, et al (2013) Bioinformatics, 29(9):1226-8) is used to detect all labeled metabolite fragments in a non-targeted manner. After quality control and ion selection, the mass isotopomer distribution (MID) is determined for all known and unknown labeled compounds. Metabolites are then sorted by their fractional contribution of tracer derived isotopes, which is correlated to their distance to the source of labeling. Using MID alignment and distance calculation, a network based on MID similarity can be constructed, which connects metabolites with similar MID patterns in the control dataset. A variability analysis is performed based on MIDs between control and treatment conditions, revealing flux changes between control and treatment conditions in a non-targeted manner. The identification of connected metabolites with variations in metabolic fluxes allows the identification of the MoA in known pathways and unknown pathways just by MID similarity.

**Methods used herein**

<u>*Tracer experiments for herbicides*</u>

*Plant material and cultivation conditions*

*Lemna paucicostata* plants were grown under sterile conditions in nutrient solution [KNO3 (400mg/L), CaCl2 * 2 H2O (540mg/L), MgSO4 * 7 H2O (614mg/L), KH2PO4 (200mg/L), Fetrilon 13% (2,81mg/L), trace element solution: MnCl2

\* 4 H2O (415mg/L), H3BO3 (500mg/L), Na2MoO4 + 2 H2O (120mg/L), ZnSO4 \* 7 H2O (50mg/L), CuSO4 \* 5 H2O (25mg/L), CoCl2 (25mg/L)] and constant light for one week prior to the experiments.

## $^{13}C\text{-}CO_2$ experiment

One-week-old *Lemna* plants were placed into nutrient solution in 30mL Falcon tubes, treated with Imazapyr (a known ALS inhibitor, 10 $\mu$M), closed with air- and liquid-permeable gauze and incubated for one hour prior to $^{13}C\text{-}CO_2$ labeling. Incubation of the plants directly in the sample tubes ensured rapid sampling times and limited the contamination with $^{12}C\text{-}CO_2$.

$^{13}C\text{-}CO_2$ labeling was done in a gas tight valve-equipped plexiglass box connected to a gas absorber. Plant samples were placed into the box, the box was closed and $^{12}C\text{-}CO_2$ was absorbed for 30 sec. Immediately after absorption, 400ppm of $^{13}C\text{-}CO_2$ were injected into the box with a syringe. In case of the unlabeled experiments, we used the same absorption conditions and then shortly opened and reclosed the box to allow $^{12}C\text{-}CO_2$ entry.

The experiments described herein used the herbicide imazapyr in one concentration, two labeling conditions and two different labeling timepoints (3 and 6 hours after $CO_2$ injection). Five replicates were down per treatment. Due to the limited space in the box and the experimental design, the experiments had to be split into several rounds; in each round untreated controls were included for direct comparison.

After the labeling time, the box was opened, and the samples quickly harvested. Therefore, the herbicide solution was poured through the gauze, the sample was slightly dried on tissue paper, closed with a lid and directly frozen in liquid nitrogen.

## Tracer experiments for fungicides

### Media and solutions

Media are prepared using commercially available malt extract (#70167, Sigma Aldrich). Malt-solution is prepared by dissolving 20 g malt extract in 1000 mL purified water and setting the pH to 6.8. Rice leaf agar is prepared by mixing 50 g of freshly frozen young rice leaves with 1000 mL purified water and pressing it through a household filter. Subsequently, 10 g of soluble starch (VWR 1.012.571.000), 2 g of baking yeast (Biolabor) and 20 g of agar-agar (granules, Becton Dickinson, 214510) were added. Both the rice leaf agar and the malt-solution media are sterilized for 15 min. at 121°C.

### Strain

*Pyricularia oryzae,* Strain J1 was used for all experiments.

### Cultivation conditions

Initially, *Pyricularia oryzae* spores are harvested by adding 10 mL sterile malt-solution (2% w/v) to rice leaf agar plates with 14-day old fungus and loosening the spores with a drigalski spatula. The malt-solution containing the fungal spores is filtered through a double layer of sterile gauze. Subsequently, the spores are cultivated in 25 mL malt-solution in 100 mL Erlenmeyer flasks (20°C, 140 rpm, aluminium foil seal).

Four days after the start of the spore incubation two Erlenmeyer flasks, each containing 25 mL spore-solution, are mixed, diluted with 50 mL malt-solution, and homogenised using an Ultra-turrax homogenizer. One mL of the homogenised spore-solution is added to 25 mL sterile malt-solution and further cultivated in 100 mL Erlenmeyer flasks (20°C, 140 rpm, aluminium foil seal).

### $^{13}C\text{-}glucose$ experiment

Seven days after the start of cultivation (spore-harvest), 12 Erlenmeyer flasks with homogenous fungal growth were selected per treatment. Two mL glucose-solution (2% w/v) were added to each flask, as well as 100 $\mu$L solution containing the active ingredient. The glucose-solution for half of the samples consisted of 100% D-glucose-$^{12}C_6$, whereas the other half contained 50 % D-glucose-$^{12}C_6$ and 50 % D-glucose-$^{13}C_6$. An overview on active ingredient solutions is given in Table 1. Control samples were treated with 100 $\mu$L DMSO (99.7%, Berndt Kraft). After subsequent incubation at 20°C and 140 rpm for two or six hours, cells were harvested as follows. Cultivation broth was vacuum-filtrated using a Büchner-funnel with filter paper. The precipitate was scraped off the filter paper into 2 mL Eppendorf tubes and immediately frozen in liquid nitrogen. Samples were stored at -80°C until analytical processing.

The 12 samples per treatment originate from $^{13}C$-labeled and non-labelled glucose solutions, two time points and three replicates.

Table 1. Overview on the active ingredients used in the MIAMI experiments with the respective concentration of the active ingredient solution applied.

| Treatment | Concentration [μM] |
|-----------|--------------------|
| Control   |                    |
| Fung1     | 1490               |
| Fung2     | 167                |

Analytics

*Sample preparation*

*Lemna paucicostata* and *Pyricularia oryzae* samples were freeze dried overnight (Christ Epsilon 2-10D,) and ground (Bead Ruptor, Omni International Inc.) prior to extraction.

An aliquot of the samples was placed in an Eppendorf tube together with a 3 mm stainless steel ball (*lemna paucicostata* 4,8 - 5,2 mg, *pyricularia oryzae* 9,5 - 10,5 mg). 350 μL water (ultrapure) and 750 μL methanol p.a. were added. Extraction was performed in a Retsch MM300 mixer mill for 3 min., 30 Hz at ambient temperature. Subsequently sample tubes were centrifuged in a Sigma 4-16KS for 10 min at 5000 rpm and 24°C and 800 μL the supernatant from each tube was transferred into individual 400 μL water containing Eppendorf tubes for extracts collection. The original tube, containing the pellet and remaining volume of the first extraction, was used for a second extraction with a mixture of 190 μL methanol and 660 μL dichloromethane p.a. Extraction and centrifugation was repeated as done in the first extraction step. 800 μL of the supernatant of this second extraction step was transferred into the water and first extract containing Eppendorf extracts collection tube, related to the respective tissue sample. It was shaken for 10 sec. (Vortex mixer) and for clear phase separation it was centrifuged as done before.

Aliquots of the upper polar layer (*lemna paucicostata* 500 μL, *pyricularia oryzae* 133 μL) and lower lipid layer (*lemna paucicostata* 50 μL, *pyricularia oryzae* 144 μL) were transferred into individual glass vials for derivatization and analysis.

Extracts were evaporated with a Genevac HT-12 evaporator centrifuge for the following derivatization steps. During the derivatization steps the vials, placed in a custom-made metal rack, have been tightly sealed by a silicon mat.

Only the lipid extract residues were trans-esterified, using a mixture of 140 μL dichloromethane, 38 μL hydrochloric acid 37% in water, 320 μL methanol and 20 μL toluene for 2 h at 100°C. Those samples were then evaporated, using a Hettlab IR Dancer infrared vortex-evaporator.

Dry lipid and polar extract residues were treated with 50 μL of O-methylhydroxylamine hydrochloride in pyridine (20 mg/mL) for 1.5 h at 60°C and MSTFA (N-Methyl-N-(trimethylsilyl)-trifluoracetamid) for 30 min. at 60°C.

Additional samples contained only aliquots of an alkane standard solution C21-C40 (No. 04071, Sigma-Aldrich) for retention index calculation.

*GC-MS analysis*

GC-MS analysis was performed using a CTC GC PAL autosampler, attached to an Agilent 6890 gas chromatograph which was coupled to an Agilent 5973 MSD mass spectrometer. 0.5 μL of the derivatized samples were injected in splitless mode at an injector temperature of 280°C. Separation was performed with helium in constant flow mode capillary columns with 30 m length, 0.25 mm i. d. and 0.25 μm film thickness. For lipid samples an Agilent HP-5MS column at 1.7 mL/min. (70°C, 50 K/min., 130°C, 10 K/min., 340°C, 9 min.) was used and for polar samples an Agilent DB-XLB column at 1.0 mL/min. (70°C, 50 K/min., 100°C, 8 K/min., 200°C, 14 K/min., 340°C, 4 min.).

Mass spectra were acquired in scan mode at 3 spec./sec. from 70 to 600 m/z.

*Computational data processing*

The generated netCDF file for each sample is imported into the MetaboliteDetector software (MetaboliteDetector - Deconvolution and Analysis of GC/MS Data; Version 3.2; http://metabolitedetector.tu-bs.de). Using the RI-calibration wizard, an n-alkane mix, specific to the day and instrument of the sample measurement, was chosen as a reference chromatogram for retention index calculation.

Next, the MetaboliteDetector files (.bin) were imported into MIA (Mass Isotopolome Analyzer; Version 1.0; (https://mia.bioinfo.nat.tu-bs.de/) for the detection of relevant metabolites. For this purpose, the labeled and unlabeled files for a respective treatment were selected and the labeled peaks determined (RI tolerance 99). Compound identification was performed using Golm Metabolome Database (http://gmd.mpimp-golm.mpg.de/) with a cutoff score of 0.75.

Subsequently, labeled metabolites were identified and the mass isotopomer distribution (MID) was calculated using the NTFD algorithm. The MID based distance calculation used the Needleman-Wunsch algorithm for gap filling and the Canberra distance measure to determine the distance between all labeled metabolites.

Subsequently, all detected metabolites were gathered in an experiment specific MetaboliteDetector compound library, containing the unlabeled spectra of all labeled metabolites, ions, the identified name, retention time and retention index.

Using MetaboliteDetectors MID wizard, the labeled files were loaded and processed using the generated library. The MIDs for all conditions using a targeted search with the non-targeted generated library were calculated.

*Variability analysis*

To identify the variability between the treatment and control the largest common ion with usable MIDs was detected. MIDs were excluded if the absolute sum of mass isotopes exceeded 1 (considering a tolerance of 0.02 for each carbon atom) (Eq 2). Ions were only used if they were present in all experimental datasets.

Equation 2

$$\sum_{i=0}^{M} |M_i| > 1 + \sum_{j=1}^{M} 0.02 \quad (2)$$

The variability between the selected common ions was the difference between the unlabeled mass isotopomer M0 of treatment and control. A threshold of 5% for polar metabolites and 2% for non-polar metabolites was used.

*Sorting*

Under the assumption that the $^{13}$C tracer distributes through the organism starting from the entry point with a decreasing fractional contribution towards more downstream metabolites, the detected metabolites can be sorted based on the total amount of labeling. The fractional contribution (FC) is the amount of labeling of the metabolite related to the number of carbon atoms M (Eq 3), where $m_i$ is the ith mass isotopomer.

Equation 3

$$FC = \frac{\sum_{i=0}^{M} m_i \cdot i}{M} \quad (3)$$

A higher FC indicates a closer proximity to the tracer than a metabolite with a lower FC.

*Context generation*

Based on fractional contribution and MID distances, pathways can be constructed without a priori biochemical knowledge. Nonetheless, the generated data needs to be set into a biochemical context. Here the identification of the detected metabolites is crucial to extrapolate the enzymatic reactions taking place.

**Results**

MIAMI-results were generated for two fungicides and one herbicides in a blind study (without the data analyst knowing the mode-of-action) as a retrospective validation of the method.

In general, MIAMI-results consist of a set of metabolites that display the largest changes in MID between treatment and control. Contextualization of these metabolites is based on their enrichment and their MID similarity, without *a priori* biochemical knowledge on the metabolite. Data interpretation is supported through library-assisted metabolite identification and biochemical pathway knowledge. In the coming paragraphs an overview on the amount of detected and changed metabolites is given, as well as the contextualized MIAMI results and the data interpretation for each of the investigated pesticides.

*Analysis of known fungicide modes-of-action*

For the known fungicides a total of 127 and 137 metabolites showed label incorporation, of which approximately 60 % were measured in the polar phase. In total, the amount of changed metabolites varied between treatments from 42 to 44 %, with most changes in the non-polar phase (table 2).

Table 2. Number of detected and changed metabolite MIDs for known fungicides.

| Fungicide | Polar metabolites detected | Polar metabolites changed | Non-polar metabolites detected | Non-polar metabolites changed |
|---|---|---|---|---|
| fung 1 | 77 | 19 | 50 | 34 |
| fung 2 | 84 | 26 | 53 | 34 |

*Ergosterol biosynthesis inhibitors: Fungicide 1 - fenpropidin and Fungicide 2 - epoxiconazole*

The similarities in labeling patterns between fungicide one and two were apparent from the beginning (Figure 2). For both treatments the metabolites showing the largest treatment effects are near-to the same (Figure 3). Therefore, both fungicides are analyzed simultaneously.

Figure 2 shows the Mass isotope distribution (MID) of two unidentified metabolites, RI 3506 and RI 3309 for fungicide one and fungicide two. The 2 h and 6 h graphs differ in incubation time after treatment. Each individual graph shows the mass distribution (M0, M1, M2, ... Mn for n = number of $^{13}$C atoms in the molecule) of the metabolite for that particular combination of treatment and incubation time point. The decrease and increase triangles visualize how the MID has changed in comparison to the respective control. Grey circles represent unchanged MIDs. The dashed line indicates the fractional contribution of the metabolite (fraction of $^{13}$C atoms in the metabolite).

In total, seven metabolites show a noticeably large differential MID (Figure 3). However, two of these metabolites can only be detected under fungicide two treatment. Meaning that although both fungicide treatments are very similar, still distinct and prominent changes are identified.

Usually, the differentially labeled metabolites are identified using a library in the next step. Unfortunately, in this case not a single metabolite of the list could be uniquely identified using the NIST library, although for all metabolites the identification could be narrowed down to ergosterol-related compounds. This is a known problem in the identification of ergosterol-related compounds due to their high degree of similarity. The possibility that different derivates of the same metabolite exist within the subset cannot be excluded.

One of the powerful features of MIAMI allows contextualization and biological interpretation of unknown metabolites. Based on MID similarity closely related metabolites can be assembled into groups that approximate pathways and ranked according to the assumed sequence of enzymatic reactions, without a priori biochemical knowledge. In the case of fungicides one and two, all differentially labeled metabolites belonged to one pathway (likely ergosterol-biosynthesis) and the sequence of reactions can be taken from Figure 3.

The metabolites displaying the largest treatment effects for fungicide one and two are listed. As none of these metabolites could be uniquely identified using the NIST data base, their retention indices (RI) are used as identifiers. The ranking (#) of the seven metabolites is based on the MID similarity and their fractional contribution (FC) in the control. The ranking represents an approximation of the in vivo sequence of enzymatic reactions. In the two most-right columns the changes in isotope distribution between treatment and control are represented graphically. Increase triangles indicate an increase in label incorporation in the metabolite under treatment, whereas red triangles indicate a decrease. For metabolites that are not found in a particular treatment, the symbol for an empty is used.

These results indicate a clear breaking point in the metabolic sequence with an enzymatic inhibition taking place between metabolites RI 3507 and RI 3309 for fungicide one and between RI 3569 and RI 3309 for fungicide two. This can be concluded because all metabolites in the sequence up until RI 3569 show an increase in label incorporation, whereas the once further down in the sequence all show a clear decrease. If both fungicides were to be analyzed separately, the next step would be to identify the metabolites RI 3507, RI 3309 and RI 3569 to pinpoint the exact enzyme that is inhibited. Such identification can be performed using other available libraries or in silico identification based on the MS-spectrum. Although improving our libraries and enabling in silico identification would be a tremendous benefit for the interpretation of these results, this was out of scope for MIAMI and will likely be followed up in a different project.

Based on both metabolite sequences two valid hypotheses can be generated on the pathway (Figure 4). In hypothesis A a linear pathway is assumed in which 3 or more enzymatic steps take place between RI 3507 and RI 3309, in which fungicide one inhibits the first enzyme and fungicide two inhibits the third. The sudden appearance of RI 3442 and RI 3569 under fungicide two treatment is then explained by an accumulation in metabolite concentration due to the proposed pathway blockage. As this would not occur in the control or the fungicide one treatment, this explains the absence of the metabolites RI 3442 and RI 3569 under those conditions.

In hypothesis B a non-linear pathway is assumed in which multiple enzymatic steps take place between RI 3507 and RI 3309, and RI 3442 and RI 3569 are produced in a side-branch of the pathway. Although according to this hypothesis both fungicides act on an enzyme between RI 3507 and RI 3309, it is sure that they do not act on the same enzyme. If they would act on the same enzyme, the appearance of RI 3442 and RI 3569 only under fungicide two treatment would not be explained. Also, because multiple time points were measured in the experiment, the time component of the treatment effect can be analyzed. For RI 3507 the fractional contribution levels in the control only vary between 0.09 and 0.12 across the time points, whereas both for fungicide one and two the levels are clearly increased between 0.16 and 0.37 (Figure 4). A closer look at the time component reveals that 2 hours after treatment both fungicides have a similar fractional contribution (0.16 and 0.17), whereas after 6 hours the enrichment of metabolite RI 3507 has increased significantly more under fungicide two treatment (0.37) than under fungicide one treatment (0.18). This points to an enzyme inhibition earlier in the pathway for fungicide two than for fungicide one. Likely, this is also linked to the overflow of metabolites into the side-branch of the pathway producing RI 3442 and RI 3569.

In Figure 4 the connecting arrows indicate one or more enzymatic steps between the metabolites. Whenever an increase in label incorporation is detected, the metabolite box is equipped with an upwards triangle. Decreases are represented by a triangle pointing downward. These triangles are placed in the upper left (fungicide one) and upper right (fungicide two) corner. Small crosses occur for metabolites that are not detectable, whereas big crosses indicate the mode-of-action hypothesis.

To assess the viability of both hypotheses, identification of the metabolites was attempted using an existing library. In this way, six of the seven metabolites could be associated to a metabolite. RI 3507 was annotated as 24-methylene-cycloartenol, RI 3309, RI3069, RI3278 and RI3219 as ergosterol derivatives. Biochemical pathway information (https://www.genome.jp/kegg/pathway.html) shows that 24-methylene-cycloartenol is indeed upstream of both fungicide targets in the phytosterol pathway, which is normally not active in fungi. The ergosterol derivatives are downstream of the targets. RI3342 matched a lipophilic metabolite of unknown identity, a so-called "known Unknown". Its unknown identity, in presence of known spectra for lanosterol, ergosterol and cycloartenol, make it more likely to represent a side product as an intermediate. Therefore, hypothesis B is more likely than hypothesis A. This is in agreement with the target of fungicide 1 being later in the pathway than that of fungicide 2.

It is clear that MIAMI has successfully pinpointed the modes-of-action of fenpropidin and epoxiconazole to a hand full of potential targets in steroid biosynthesis. As both of these fungicides have a known mode-of-action within this subset of potential targets, this result provides a retrospective validation of MIAMI.

In the present use case (mode-of-action identification of fungicides) MIAMI showed a greater potential to elucidate the mode-of-action as compared to metabolomics. Metabolomics results on four time points for fenpropidin show reductions in ergosterol, however not significantly after 6 hours of treatment (Figure 5). With MIAMI we can show significant differences already after 2 hours of treatment. This indicates that MIAMI is the method of choice for fast-acting pesticides as it captures effects earlier after treatment. Metabolomics can pinpoint the mode-of-action at the 48-hour time point to ergosterol biosynthesis, which means that still approximately 40 enzymes are potentially inhibited, whereas MIAMI could narrow this down to a hand full of enzymes due to its unique contextualization feature. This also enables MIAMI to analyze and interpret metabolites that were previously unknown. As the analytical method is untargeted, it is also clear that potentially more metabolites are captured, increasing the coverage of metabolic pathways and therefore increasing the likelihood of discovering new modes-of-action.

*Analysis of known herbicides modes-of-action*

For the known herbicide a total of 110 metabolites showed label incorporation, of which approximately 90 % were measured in the polar phase. The low number of detected metabolites in the non-polar phase is very likely due to a low $^{13}$C-incorporation into these metabolites. Unfortunately, this decreases the pathway coverage significantly, and with that, also the potential to discover a mode-of-action drops noticeably. In total, the amount of changed metabolites was 46%.

<u>Acetolactate synthase inhibitor: Herbicide 4 - Imazapyr</u>

Overall the label incorporation was very small, although present, for many metabolites after herbicide four treatment. All differentially labeled metabolites displayed an increase in fractional contribution, except for valine (Figure 6). This indicates a mode-of-action in the valine biosynthesis pathway (Figure 7), however to be more accurate on which enzyme is inhibited specifically, a higher coverage is needed. The limited resolution for herbicide four is definitely due to the lack of label incorporation for this treatment, because of which less metabolites could be detected, decreasing the coverage significantly. Nonetheless, MIAMI identified a potential mode-of-action in the branched-chain amino acid synthesis pathway. This is correct, however doesn't yield a satisfactory result as a specific enzyme could not be identified.

In Figure 6, each individual graph shows the mass distribution (M0, M1, M2, ... Mn for n = number of $^{13}$C atoms in the molecule) of the metabolite for that particular combination of treatment and incubation time point. The decrease and increase triangles visualize how the MID has changed in comparison to the respective control. Grey circles represent unchanged MIDs. The dashed line indicates the fractional contribution of the metabolite (fraction of $^{13}$C atoms in the metabolite).

**Claims**

1. A method of characterising the mode of action of a test compound comprising

    a) A first biological sample and a second biological sample, wherein there is at least one isotopic difference between the samples, and wherein the first and second biological samples are isolated from a living system exposed to the test compound;
    b) A third biological sample and a fourth biological sample, wherein there is at least one isotopic difference between the samples, and wherein the third and fourth biological samples are isolated from a living system not exposed to the test compound;
    c) Performing chromatograph and mass spectrometry analysis of metabolites extracted from the biological samples to provide mass spectra data;
    d) Calculating the mass isotopomer distribution for the metabolites from the mass spectra;
    e) Identifying metabolites that have a significant different isotopomer distribution when the living system was exposed to the test compound to the isotopomer distribution when the living system was not exposed to the test compound.

2. The method of claim 1 wherein the mass spectra data provided in step c) is deconvoluted and metabolites subsequently identified from the mass spectra by data comparison with a reference library.

3. The method of claim 1 or 2 wherein the mass isotopomer distribution generated by step d) is determined by: calculating the retention indices and deconvolution of the mass spectral data; pairing the deconvoluted mass spectra data across samples and identifying metabolites according to isotope incorporation; identification of those metabolites based on similarity of spectra, retention time and/or retention index by comparison to a reference library; calculation of the mass isotopomer distribution of the metabolites.

4. The method of any of the previous claims wherein step e) comprises comparing the mass isotopomer distribution between metabolites having at least one isotopic difference and identifying those metabolites which have a statistically different deviation between the biological samples isolated from a living system exposed to the test compound and the biological samples isolated from a living system not exposed to the test compound.

5. The method of any of the previous claims wherein the mode of action of the test compound is identified according to the metabolites identified from step e).

6. The method of any of the previous claims wherein living system incorporates an isotopic label from an isotope-labeled substrate.

7. The method of claim 6 wherein the isotope-labeled substrate is chosen from $^2H_2O$, $H_2\ ^{18}O$, $^2H$-glucose, $^2H$-labeled amino acids, $^2H$-labeled organic molecules, $^{13}C$-labeled organic molecules, $^{13}C$-labeled glycerol, $^{13}CO_2$, $^{15}N$-labeled organic molecules, $^3H_2O$, $^3H$-labeled glucose, $^3H$-labeled amino acids, $^3H$-labeled organic molecules.

8. The method of any of the previous claims wherein the living system is a plant, fungus, virus, bacteria, invertebrate or vertebrate.

9. The method of any of the previous claims wherein the living system is a plant and the isotope-labeled substrate is $^{13}CO_2$.

10. The method of any of claims 1 to 8 wherein the living system is a fungus and the isotope-labeled substrate is $^{13}C$-labeled organic molecules.

11. The method of any of the previous claims wherein the living system is exposed to the isotope-labeled substrate for between 10 mins and 48 hours.

12. The method of any of the previous claims wherein the living system is exposed to the test substrate for between 10 mins and 48 hours.

**Patentansprüche**

1. Verfahren zur Charakterisierung der Wirkungsweise einer Testverbindung, umfassend

   a) eine erste biologische Probe und eine zweite biologische Probe, wobei es mindestens einen Isotopenunterschied zwischen den Proben gibt und wobei die erste und die zweite biologische Probe aus einem lebenden System isoliert werden, das der Testverbindung ausgesetzt ist;
   b) eine dritte biologische Probe und eine vierte biologische Probe, wobei es mindestens einen Isotopenunterschied zwischen den Proben gibt und wobei die dritte und die vierte biologische Probe aus einem lebenden System isoliert werden, das nicht der Testverbindung ausgesetzt ist;
   c) die Durchführung einer Chromatographie- und Massenspektrometrie-Analyse von Metaboliten, die aus den biologischen Proben extrahiert wurden, unter Bereitstellung von Massenspektraldaten;
   d) das Berechnen der Massenisotopomerenverteilung für die Metaboliten aus den Massenspektren;
   e) die Identifizierung von Metaboliten, die eine signifikant unterschiedliche Isotopomerverteilung aufweisen, wenn das lebende System der Testverbindung ausgesetzt wurde, im Vergleich zu der Isotopomerverteilung, wenn das lebende System nicht der Testverbindung ausgesetzt wurde.

2. Verfahren nach Anspruch 1, wobei die in Schritt c) bereitgestellten Massenspektrumdaten dekonvolutiert werden und anschließend durch Datenvergleich mit einer Referenzbibliothek Metaboliten aus den Massenspektren identifiziert werden.

3. Verfahren nach Anspruch 1 oder 2, wobei die durch Schritt d) erzeugte Massenisotopomerverteilung bestimmt wird durch: Berechnen der Retentionsindizes und Dekonvolutierung der Massenspektraldaten; Paaren der dekonvolutierten Massenspektraldaten über Proben und Identifizieren von Metaboliten gemäß Isotopeneinbau; Identifizieren dieser Metaboliten basierend auf der Ähnlichkeit von Spektren, Retentionszeit und/oder Retentionsindex durch Vergleich mit einer Referenzbibliothek; Berechnen der Massenisotopomerverteilung der Metaboliten.

4. Verfahren nach einem der vorhergehenden Ansprüche, wobei Schritt e) das Vergleichen der Massenisotopomerverteilung zwischen Metaboliten mit mindestens einem Isotopenunterschied und das Identifizieren derjenigen Metaboliten, die eine statistisch unterschiedliche Abweichung zwischen den biologischen Proben, die aus einem der Testverbindung ausgesetzten lebenden System isoliert wurden, und den biologischen Proben, die aus einem der Testverbindung nicht ausgesetzten lebenden System isoliert wurden, aufweisen, umfasst.

5. Verfahren nach einem der vorhergehenden Ansprüche, wobei die Wirkungsweise der Testverbindung gemäß den aus Schritt e) identifizierten Metaboliten identifiziert wird.

6. Verfahren nach einem der vorhergehenden Ansprüche, wobei das lebende System eine Isotopenmarkierung von einem isotopenmarkierten Substrat beinhaltet.

7. Verfahren nach Anspruch 6, wobei das isotopenmarkierte Substrat aus $^2H_2O$, $H_2^{18}O$, $^2H$-Glucose, $^2H$-markierten Aminosäuren, $^2H$-markierten organischen Molekülen, $^{13}C$-markierten organischen Molekülen, $^{13}C$-markiertem Glycerin, $^{13}CO_2$, $^{15}N$-markierten organischen Molekülen, $^3H_2O$, $^3H$-markierter Glucose, $^3H$-markierten Aminosäuren und $^3H$-markierten organischen Molekülen ausgewählt ist.

8. Verfahren nach einem der vorhergehenden Ansprüche, wobei das lebende System eine Pflanze, ein Pilz, ein Virus, Bakterien, ein wirbelloses Tier oder ein Wirbeltier ist.

9. Verfahren nach einem der vorhergehenden Ansprüche, wobei das lebende System eine Pflanze ist und das isotopenmarkierte Substrat $^{13}CO_2$ ist.

10. Verfahren nach einem der Ansprüche 1 bis 8, wobei das lebende System ein Pilz ist und das isotopenmarkierte Substrat ein $^{13}C$-markiertes organisches Molekül ist.

11. Verfahren nach einem der vorhergehenden Ansprüche, wobei das lebende System dem isotopenmarkierten Substrat 10 min bis 48 h ausgesetzt wird.

12. Verfahren nach einem der vorhergehenden Ansprüche, wobei das lebende System dem Testsubstrat 10 min bis 48 h ausgesetzt wird.

**Revendications**

1. Procédé de caractérisation du mode d'action d'un composé d'essai comprenant

   a) un premier échantillon biologique et un deuxième échantillon biologique, les échantillons présentant au moins une différence isotopique, et les premier et deuxième échantillons biologiques étant isolés à partir d'un système vivant exposé au composé d'essai ;
   b) un troisième échantillon biologique et un quatrième échantillon biologique, les échantillons présentant au moins une différence isotopique, et les troisième et quatrième échantillons biologiques étant isolés à partir d'un système vivant non exposé au composé d'essai ;
   c) la réalisation d'une analyse par chromatographie et spectrométrie de masse des métabolites extraits à partir des échantillons biologiques afin d'obtenir des données de spectres de masse ;
   d) le calcul de la distribution d'isotopomères massiques pour les métabolites à partir des spectres de masse ;
   e) l'identification des métabolites qui présentent une distribution d'isotopomères significativement différente lorsque le système vivant a été exposé au composé d'essai par rapport à la distribution d'isotopomères lorsque le système vivant n'a pas été exposé au composé d'essai.

2. Procédé selon la revendication 1, dans lequel les données de spectres de masse obtenues à l'étape c) sont déconvoluées et les métabolites consécutivement identifiés à partir de ces spectres par comparaison des données à une bibliothèque de référence.

3. Procédé selon la revendication 1 ou 2, dans lequel la distribution d'isotopomères massiques générée à l'étape d) est déterminée par : calcul des indices de rétention et déconvolution des données de spectres de masse ; appariement des données de spectres de masse déconvoluées entre les échantillons et identification des métabolites en fonction de l'incorporation isotopique ; identification de ces métabolites sur la base de la similarité des spectres, du temps de rétention et/ou de l'indice de rétention par comparaison à une bibliothèque de référence ; calcul de la distribution d'isotopomères massiques des métabolites.

4. Procédé selon l'une quelconque des revendications précédentes, dans lequel l'étape e) comprend la comparaison de la distribution d'isotopomères massiques entre les métabolites présentant au moins une différence isotopique et l'identification des métabolites qui présentent un écart statistiquement différent entre les échantillons biologiques isolés à partir d'un système vivant exposé au composé d'essai et les échantillons biologiques isolés à partir d'un système vivant non exposé au composé d'essai.

5. Procédé selon l'une quelconque des revendications précédentes, dans lequel le mode d'action du composé d'essai est identifié en fonction des métabolites identifiés à l'étape e).

6. Procédé selon l'une quelconque des revendications précédentes, dans lequel le système vivant incorpore un marqueur isotopique provenant d'un substrat marqué par un isotope.

7. Procédé selon la revendication 6, dans lequel le substrat marqué par un isotope est choisi parmi $^2H_2O$, $H_2^{18}O$, $^2H$-glucose, acides aminés marqués par $^2H$, molécules organiques marquées par $^2H$, molécules organiques marquées par $^{13}C$, glycérol marqué par $^{13}C$, $^{13}CO_2$, molécules organiques marquées par $^{15}N$, $^3H_2O$, glucose marqué par $^3H$, acides aminés marqués par $^3H$, molécules organiques marquées par $^3H$.

8. Procédé selon l'une quelconque des revendications précédentes, dans lequel le système vivant est une plante, un champignon, un virus, une bactérie, un invertébré ou un vertébré.

9. Procédé selon l'une quelconque des revendications précédentes, dans lequel le système vivant est une plante et le substrat marqué par un isotope est $^{13}CO_2$.

10. Procédé selon l'une quelconque des revendications 1 à 8, dans lequel le système vivant est un champignon et le substrat marqué par un isotope est constitué de molécules organiques marquées par $^{13}C$.

11. Procédé selon l'une quelconque des revendications précédentes, dans lequel le système vivant est exposé au substrat marqué par un isotope pendant 10 minutes à 48 heures.

12. Procédé selon l'une quelconque des revendications précédentes, dans lequel le système vivant est exposé au substrat d'essai pendant 10 minutes à 48 heures.

Figure 1

Figure 2

A

B

# Figure 2

**C**

fung1_2h_13c

**D**

fung1_6h_13c

Figure 2

**E**

**F**

Figure 2

**G**

**H**

Figure 2

I

J

# Figure 2

**K**

**L**

## Figure 2

**M**

**N**

Figure 2

O

P

Figure 3

| # | Metabolite | FC | Fung 1 | Fung 2 |
|---|---|---|---|---|
| 1 | RI 3507.33 | 0.37 | ▲ | ▲ |
| 2 | RI 3442.33 | 0.14 | ∅ | ▲ |
| 3 | RI 3569.35 | 0.13 | ∅ | ▲ |
| 4 | RI 3309.47 | 0.01 | ▼ | ▼ |
| 5 | RI 3068.89 | 0.01 | ▼ | ▼ |
| 6 | RI 3278.05 | 0.01 | ▼ | ▼ |
| 7 | RI 3219.20 | 0.01 | ▼ | ▼ |

Figure 4

Figure 5

# Fenpropidin treatment

| | 0.5 h | 2 h | 6 h | 48 h |
|---|---|---|---|---|
| Lanosterol, free | 1.87 | 1.55 | 2.07 | 1.93 |
| Lanosterol, total | 0.79 | 0.86 | 1.01 | 3.00 |
| Ergosterol, free | 1.13 | 0.96 | 0.93 | 0.60 |
| Ergosterol, total | 1.07 | 0.78 | 0.95 | 0.39 |
| Ergosterolester No 01 | 0.99 | 1.02 | 0.99 | 0.26 |
| 22,23 Dihydroergosterolester No 01 | 0.99 | 0.87 | 0.75 | 0.10 |
| Unknown lipid (62100097) | 17.99 | 12.19 | 16.04 | 26.72 |
| Unknown lipid (62100014) | 1.61 | 2.26 | 2.06 | 1.99 |
| Unknown lipid (62100093) | 0.40 | 0.38 | 0.48 | 0.16 |

Figure 6

A

B

Figure 6

C

D

# Figure 6

**E**

**F**

Figure 6

G

H

Figure 7

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2005051434 A1

**Non-patent literature cited in the description**

- **WEINDL et al.** *Bioinformatics*, 2016, vol. 32 (18), 2875-2876

- **HILLER K et al.** *Bioinformatics*, 2013, vol. 29 (9), 1226-8